# EUROPEAN PATENT APPLICATION

(11) **EP 2 813 149 A1**
(43) Date of publication of application: **17.12.2014**
(21) Application number: 13171984.1
(22) Date of filing: 14.06.2013
(51) Int. Cl.: A23L 1/29, A23L 1/30, A23L 1/305, A23L 1/308, A61P 3/02, A61P 25/08, A61P 25/28, A61K 31/00

(54) **Dietetic compositions for the treatment of malnutrition, neurological diseases and metabolic diseases**

(71) Applicant: Nestec S.A., 1800 Vevey (CH)
(72) Inventor: The designation of the inventor has not yet been filed
(74) Representative: Stephen, Paula-Marie

(57) **Abstract**

The present invention is directed to compositions, preferably dietetic compositions suitable for the treatment of malnutrition, in the treatment of neurological diseases, particularly epilepsy and related diseases such as difficult-to-control (refractory) epilepsy in children, and in the treatment of metabolic diseases or other diseases. The inventive compositions as detailed herein specifically provide novel ketogenic diets and compositions suitable therefore. Further inventive compositions as detailed herein are also suitable for use in dietary modification or manipulation for therapeutic effect and benefit. The present invention also describes a method for preparing such compositions and the use of such compositions.

## Description

### Field of the Invention

The present invention is directed to compositions, preferably dietetic compositions suitable for the treatment of malnutrition, in the treatment of neurological diseases, particularly epilepsy and related diseases such as difficult-to-control (refractory) epilepsy in children, and in the treatment of metabolic diseases or other diseases. The inventive compositions as detailed herein specifically provide novel ketogenic diets and compositions suitable therefore. Further inventive compositions as detailed herein are also suitable for use in dietary modification or manipulation involving the intake of fat and protein, in isolation or combination, for therapeutic effect and benefit. The present invention also describes a method for preparing such compositions and the use of such compositions.

### Background

Dietetic compositions usually aim to provide an adequate delivery of nutrients, e.g. of a specific distribution of fat, carbohydrates and lipids, and thereby try to reduce negative effects of e.g. malnutrition and or specific diseases. Among such dietetic compositions, a particularly well known form is the ketogenic diet (KD), specifically in the treatment of the neurological disease epilepsy. In this regard, the ketogenic diet has been used for more than 80 years for the treatment of medically intractable epilepsy. Such a ketogenic diet, as typically provided in the art, is a high-fat, low-carbohydrate, adequate-protein diet (see e.g. Carl E. Stafstrom, MD, PhD, Jong M. Rho, MD, Epilepsy and the Ketogenic Diet, Humana Press, 2004, eISBN 1-59259-808-0).

The ketogenic diet (KD) was originally formulated to produce the biochemical changes ordinarily associated with fasting, a condition known since antiquity to reduce seizure activity. In this context, KD is designed to mimic the fasting state, in which the brain switches from oxidation of carbohydrates to fats as its primary energy source. Somehow, this metabolic transition is associated with improved seizure control.

Despite the diet's use for many years, fundamental questions still remain about how the KD works, how to formulate and administer the diet for optimal success, and how to choose which patients will respond best to its implementation. In this context Darryl C. de Vivo et al. found that "The rationale for the ketogenic diet is as follows: the brain derives most of its energy from the aerobic oxidation of glucose. Under certain conditions, such as fasting or consumption of high-fat diet, the brain can use ketone bodies for energy. The ketogenic diet, which is high in fat and low in carbohydrates and protein (usually in a 3: 1 or 4: 1 ratio by weight), produces a state of ketosis that mimics the fasting state" (see Epi-lepsia 39(11), p.1216-1225 (1998)), Darryl C. de Vivo et *al.,* 1^{st} paragraph, 2nd col., p.1220*)*.

The KD has become a significant feature of many epilepsy treatment programs. Although there is no longer much question that the KD has beneficial effects in a substantial proportion of drug-resistant epilepsies, there remain a large number of uncertainties regarding its application and mechanism(s) of action. These questions appear at both the clinical treatment level and in the basic research laboratory. Hence, there is still a need to better understand the KD, what it does, when, and how, and to provide dietetic compositions, which address the needs of a ketogenic diet and efficiently allow to treat epilepsy, particularly in older children and adults (see e.g. Carl E. Stafstrom, MD, PhD, Jong M. Rho, MD, Epilepsy and the Ketogenic Diet, Humana Press, 2004, eISBN 1-59259-808-0).

The traditional ketogenic diet typically employs fat to carbohydrate to protein in a ratio of 4:1:1 (see Grath G et al., in Seizure: the journal of the British Epilepsy Association, Dietary practices and use of the ketogenic diet in the UK (2000), Vol. 9, No. 2, pp. 128-30). Theoretically for the provision of sufficient energy to the brain, only the fat prescription is necessary. This is illustrated by the fact that during starvation or fasting, when intakes of carbohydrate, protein, and fat are nil, adequate amounts of energy substrates for use by the central nervous system are provided through the breakdown of fat via gluconeogenesis (glycerol) and ketogenesis (ketone bodies), resulting in the state of ketosis (Cahill GF (1970), Starvation in man. N Engl J Med. 282 (12):668-75). Protein requirement is prescribed on an individual patient basis, typically 0.8-1.5g/kg body weight, to ensure a supply of essential amino acids for growth and the maintenance of body tissues. The remainder of this part of the dietary prescription is given as carbohydrate to improve the palatability of the diet.

Despite such classical ketogenic diets, alternative medium chain triglyceride (MCT) diets have been tested. In the aforementioned trials such a diet employed 60% MCT fat (see Grath et al, 2000, supra). In this regard, it is also well known that the molecular weight of MCTs is smaller than the molecular size of long-chain triglycerides (LCTs). This facilitates the action of pancreatic lipase and, consequently, MCTs are hydrolyzed both faster and more completely than LCTs. This essential difference between MCTs and LCTs forms basis for many MCT based diets.

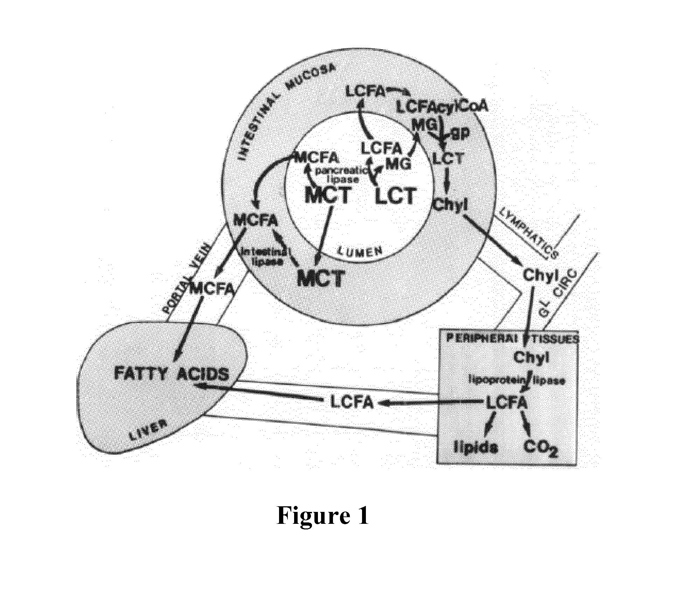

In this context, the metabolism of LCTs and MCTs plays a critical role. MCTs may be metabolized to medium-chain fatty acids (MCFA) by pancreatic lipase. LCTs are typically processed to long-chain fatty acids ( LCFA), monoacylglycerol (MG) and further metabolic products. In this context, it is well known that MCFAs follow the portal venous system, whereas LCFAs follow the lymphatic system. Thus, MCTs do not stimulate the flow of lymph, while LCTs stimulate it significantly (see André C Bach, ScD and Vigen K Ba-bayan, PhD; The American Journal of Clinical Nutrition; Medium-chain triglycerides: an update, 36: NOVEMBER 1982, pp 950-962): See Figure 1.

Interestingly, when LCTs and MCTs are ingested simultaneously, the latter partially inhibits the absorption of the former. Nevertheless, the total number of calories absorbed in this situation is greater than the calories absorbed when either fat is ingested alone. Furthermore, the absorption of calcium and magnesium appears to be enhanced when the diet contains MCTs.

Of particular interest for the ketogenic diet is the fact that when MCTs are supplied in the diet, they are rapidly oxidized, rendering many ketone bodies and supplying a quick source of energy. Accordingly, MCTs are a highly useful food of choice for any organism that has increased energy needs.

In comparison to LCT, MCT has the following properties meaning it is digested and transported differently and more quickly by the body. It has a greater solubility in water and biological fluids and a smaller molecular size. It diffuses directly from the gastrointestinal tract to the portal rather than the lymphatic system. There is no requirement for intraluminal lipases or bile salts for assimilation. It can be hydrolysed by lipase in entero-cytes with no need for incorporation into chylomicrons and it passes into the bloodstream directly, reaching the liver more rapidly than LCT, then enters the hepatic mitochondria to be metabolised via a specific pathway for MCT, independent of the carnitine cycle.

Acetyl-CoA and ketone bodies are then rapidly produced and available for use as an energy source throughout the body. This property of MCT, i.e. the greater ability to generate more ketones per unit of energy compared to LCT makes it generally very useful in the ketogenic diet. The inclusion of a proportion of the total fat as MCT also typically enhances the production of ketones and level of ketosis achieved.

The severe carbohydrate restrictions of the classic version of the ketogenic diet made it difficult for parents to produce palatable meals that their children would tolerate. In 1971, Peter Huttenlocher devised a ketogenic diet where about 60% of the calories came from the MCT oil, and this allowed more protein and up to three times as much carbohydrate as the classic ketogenic diet. Today, the level of MCT used is generally about 50% of total energy (25% LCT, 10-15% protein, 10-15% carbohydrate). A recent trial has meanwhile shown that this version of the ketogenic diet is as effective as the traditional classical version (see e.g. Neal EG et al., Lancet Neurol. 2008 Jun; 7(6):500-6; and Kossoff et al., Epi-lepsia. 2009 Feb;50(2):304-17).

However, even though there are already many approaches in the prior art which address the needs of a ketogenic diet, many of such compositions are only suitable for a small group of patients, such as infants (see e.g. EP 2 073 647) or have only shown to provide an effect for treatment of an extremely limited number of diseases. Accordingly, it is an object of the present invention, to further improve dietetic compositions which specifically address the needs of a ketogenic diet, but which also allow treatment of further diseases, which may be cured or at least ameliorated by supplemental or full nutritional feeding.

### Description of the Invention

As described herein, the object underlying the present invention is preferably to be accomplished by means of the independent claims as attached. The dependent claims advantageously illustrate further preferred aspects of the inventive embodiments.

According to a first embodiment, the object underlying the present invention is preferably solved by a composition, preferably a ketogenic composition, which typically comprises about 4 to about 12% by weight protein, less than 5% by weight carbohydrate and about 25 to about 38% by weight fat. The inventive composition may be used for treatment of diseases and disorders as defined herein.

According to a preferred aspect the composition of the present invention is a nutritional composition, preferably a pasteurized composition, more preferably a pasteurized nutritional composition. Additionally or alternatively the composition of the present invention may be a ketogenic composition. Furthermore, the composition of the present invention may be for use as a supplement or may be used as a sole source of nutrition, e.g. as a full meal. The inventive composition may be furthermore suitable for use in the treatment of a disease as defined herein.

The term "supplement" as used herein refers to a nutrient that may be added to the diet or a meal thereof.

As defined above, the inventive composition typically comprises about 4 to about 12% by weight protein, less than 5% by weight carbohydrate and about 25 to about 38% by weight fat.

Advantageously the invention composition, with a content of less than 5% by weight of carbohydrate makes it possible to have higher protein content compared to classical and MCT ketogenic diets, at a same ratio of fat to the sum of proteins and carbohydrates. Within a ketogenic diet, carbohydrate as a source of glucose is not an essential constituent, from a nutritional perspective, provided adequate and appropriate amounts of fat and protein are ingested to meet energy requirements, so the body can manufacture sufficient alternative energy substrates from these instead. Whereas protein intakes can be inadequate for some patients on a ketogenic diet the invention composition allows to provide a more adequate protein intake by increasing the amount of protein that can be given within a defined ketogenic ratio.

Within the context of the present invention, the protein, the fat and/or the carbohydrate, where present, of the inventive composition is/are preferably derived from at least two different sources, namely animal and vegetal. For example, the proteins are derived at least partly from animal, such as whey protein, but optionally also partly from plant source, the fat at least partly from vegetal or plant source, and the carbohydrates at least partly from vegetal source, or from a combination of animal and vegetal. Likewise, if at least two preferably different types of fat, at least two preferably different types of protein and/or at least two preferably different types of carbohydrate is/are contained in the inventive composition, such proteins, fats and/or carbohydrates of the inventive composition may be derived from at least two different sources, namely animal and vegetal.

Unless otherwise indicated, the term "at least" in the context of the present invention typically preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

According to one preferred aspect, the protein as contained in the inventive composition typically may be selected from any suitable protein selected from the group comprising or consisting of e.g. whey protein, whey protein isolate, preferably acidified whey protein isolate, whey protein concentrate, whey powder, egg protein, pea protein, potato protein, soy protein, soy protein isolate, and combinations thereof and the like either alone or in combination. In a preferred embodiment the composition does not contain whole milk protein in a ratio of 80:20 casein protein to whey protein.

It is also preferable that intact proteins are used in the inventive composition. It is further preferred that casein is not present in the inventive composition as described herein. For purposes of the present invention, the composition as defined herein preferably comprises about 4 to about 12% by weight protein, preferably about 4 to about 11 % by weight protein, likewise preferably about 4 to about 10%, or about 5 to about 10%, or about 5 to about 7%, or about 6 to about 8%, or about 7.5 to about 9%, or about 8.5 to about 10%. The present invention most preferably has a protein content of about 8 % by weight, which is particularly preferably acidified whey protein isolate.

Furthermore, according to one preferred aspect, the fat as contained in the inventive composition typically may be selected from any suitable fat source, selected from the group comprising or consisting of coconut oil, preferably fractionated coconut oil (MCT), lemon oil, dietary fats, vegetable oil, such as sunflower oil, preferably high oleic sunflower oil, canola oil, corn oil, soybean oil, sesame seed oil, safflower oil, walnut oil, evening primrose oil, peanut oil, cottonseed oil, rapeseed oil, algal oil, olive oil, fish oil (e.g., menhaden oil, sardine oil) macadamia oil, palm oil, palm kernel oil, or mixtures thereof, etc., either alone or in combination. According to the present invention, dietary fats are preferably selected from (fat) molecules composed of individual carbon atoms linked into chains ranging from 2 to 24 carbon atoms in length. Preferably, the inventive composition does not comprise soy protein and/or olive oil and/or canula oil.

The fat source used in the inventive composition can be a 100% LCT or 100% MCT fat source, which can be employed either alone or in combination. Accordingly, the inventive compositions may comprise a mixture of MCT and LCT as defined herein. Alternatively, if for instance solely a MCT fat source is utilized, this provides compositions which may lack an LCT component. If for instance solely a LCT fat source is utilized, this provides compositions which may lack an MCT component.

For the purposes of the present invention, the composition as defined herein preferably comprises about 25 to about 38% by weight fat, more preferably about 28 to about 35% by weight fat, or about 26 to about 28%, or about 27 to about 28.5%, or about 27.5 to about 30%, or about 29 to about 31%, or about 30.5 to about 32 %, or about 31.5 to about 33.5 %, or about 33 to about 34.5%, or about 34 to about 36%, or about 35 to about 37% by weight fat. According to one preferred embodiment the composition has a fat content of about 32% to about 34% by weight.

Within the context of the present invention, the inventive composition may also comprise or contain fat comprising saturates, mono-unsaturates, and/or poly-unsaturates. Preferably, the inventive composition preferably comprises in a range of about 2 to about 30% by weight saturates, about 1 to about 25% by weight mono-unsaturates and about 0.1 to about 5% by weight poly-unsaturates.

Particularly preferable, the saturated fat is present in the inventive composition in a range of about 2 to about 30% by weight, preferably about 17 to about 19.5%, or about 19 to about 21%, or about 20 to about 22%, or about 21 to about 25%, or about 24 to about 29% by weight. Most preferably, the present invention has saturated fat present at about 20.97% by weight. The term "%" is preferably defined as "% by weight" and in this context reflects the total amount of the saturated fat component in the inventive composition.

Likewise preferable, the mono-unsaturated fat is present in the inventive composition in a range of about 1 to about 25% by weight, preferably about 2 to about 4%, or about 3 to about 5%, or about 4.5 to about 6%, or about 5.5 to about 6.7%, or about 6.5 to about 7%, or about 6.8 to about 10% by weight. Most preferably, the present invention has monoun-saturated fat present at about 6.68% by weight. The term "%" is preferably defined as "% by weight" and in this context reflects the total amount of the mono-saturated fat component in the inventive composition.

Similarly, it is preferred that the poly-unsaturated fat is present in the inventive composition in the range of about 0.1 to about 5% by weight, preferably about 0.3 to about 0.8% or about 0.6 to about 1%, or about 0.9 to about 1.2%, or about 1 to about 1.3%, or about 1.1 to about 1.5, or about 1.3 to about 2% by weight. Most preferably, the present invention has polyunsaturated fat present at about 1.2% by weight. The term "%" is preferably defined as "% by weight" and in this context reflects the total amount of the poly-saturated fat component in the inventive composition.

According to one further preferred aspect, the inventive composition comprises or contains an amount about 0 to about 23% by weight medium chain triglycerides and/or an amount of about 6 to about 35 % by weight long chain triglycerides.

Medium Chain Triglycerides (MCTs) as used herein are typically composed of 6 to 10, or 6 to 11, or 6 to 12 carbon links. Because of their shorter chain length MCTs have a number of unique properties which give them advantages over the more common LCTs. The MCT's employed in the present invention are preferably sourced from fractionated coconut oil, macadamia oil, palm oil, palm kernel oil, etc. and combinations thereof.

The medium chain triglycerides which may be present in the inventive composition typically range from about 0 to about 23% by weight. In one preferred embodiment the inventive composition may be substantially free of MCTs. In some preferred embodiments the inventive composition comprises no more than 0.5% by weight, or about 0.001% to about 0.5% by weight MCTs In another preferred embodiment the composition comprises about 0 to about 22% by weight, such as about 0.5 to about 22% by weight. preferably about 12 to about 16% or about 14 to about 20%, or about 18 to about 22% by weight MCTs.

Long Chain Fatty acids (LCTs) as used herein ranging from 12 to 18 carbons long are the predominant form of fat in the human diet. Fatty acids resulting from the breakdown of LCT are a major fuel source for most body tissues. Their oxidation plays an important role in maintaining energy homoeostasis, particularly for the brain, during periods of fasting. LCT's employed in the present invention are preferably sourced from sunflower oil, most preferably high oleic sunflower oil, corn oil, soy oil, canola oil, sesame seed oil, safflower oil, walnut oil, evening primrose oil, peanut oil, cottonseed oil, rapeseed oil, olive oil, fish oil (e.g., menhaden oil, sardine oil), palm olein etc. and combinations thereof.

The long chain triglycerides which may be present in the inventive composition typically range from about 6 to about 38 % by weight. In one preferred embodiment solely a LCT fat source is used. In one preferred embodiment LCTs are present in the inventive composition from about 25 to about 38% by weight, preferably from about 28 to about 35% by weight, more preferably from about 30 to about 34% by weight. In one preferred, the present invention has Long chain triglycerides present at about 33% by weight. In some embodiments LCTs are present in the inventive composition from about 7 to about 8%, or about 7.5 to about 8.7%, or about 8 to about 10%, or about 9 to about 15% by weight.

Additionally, according to one other preferred aspect, the carbohydrate source as contained in the inventive composition typically may be selected from any suitable carbohydrate source. According to some embodiments, the carbohydrate source employed in the inventive composition may include sucrose, preferably castor sugar, fructose, maltodextrin, fibers, corn syrup, high fructose corn syrup, corn starch, lactose, glucose, dextrose, maltose, and the like, either alone or in combination.

For the purposes of the inventive composition less than 5% by weight carbohydrate is employed, preferably from 0 to about 4.5% by weight, more preferably from 0 to about 4% by weight or about 0.05 to about 4.5% by weight, or about 0.05 to about 4% by weight. In some preferred embodiments from 0 to about 3% by weight carbohydrate is employed, or about 0.05 to about 3%, preferably from 0 to about 2%, or about 0.05 to about 2%, more preferably 0 to about 1%, or about 0.05 to about 1%. The present invention most preferably has a carbohydrate content of less than 1% by weight, preferably less than 0.5% by weight, such as about 0.1 % by weight.

For the purposes of the inventive composition, sweetener may also be present, which may be a combination of a high dextrose equivalent sweetener, an artificial sweetener and a highly concentrated sweetener. For instance, the sweetener or combination of sweeteners may be crystalline fructose, aspartame, corn syrup, sucrose, glucose, sucralose, maltodextrin, and/or stevia.

Sweeteners include, but are not limited to, dextrose, crystalline fructose, high fructose corn syrup 90%, high fructose corn syrup 55%, and high fructose corn syrup 42%. Other complementary sweeteners that may also be used in the optional sweetener blend include, but are not limited to fructose, sucrose, glucose, lactose, maltose, dextrose, honey, invert sugar, corn syrup, rice syrup, grain syrup, maltodextrin, polydextrose, oligodextrin, cane based sweeteners, beet based sweeteners etc. Optionally, artificial sweeteners, sugar substitutes and non-nutritive sweeteners may also be used and include, but are not limited to, edible saccharin salts, aspartame, saccharine, alitame, acesulfame K, tagatose, L-sugars, neophesperidin, thaumatin, dihydrochalchones, cyclamates, steviosides, glycyrrhizins, synthetic alkoxy aromatics, such as dulcin, sucralose, suosan, miraculin, monellin, sorbitol, xylitol, talin, cyclohexylsulfamates, synthetic sulfamic acids, oximes, aspartyl malo-nates, succanilic acids, amino acid based sweeteners, and carboxylates such as 3-hydroxy-4-alkyloxyphenyl aliphatic carboxylates.

Said optional sweetener blend is preferably selected according to the desired sweetness. For instance, it is well known that fructose in its pure crystalline form has a perceived sweetness of about 160 to 180, typically reported as 173, compared to sucrose at a value of 100. Hence, if the optional sweetener blend comprises fructose, other sweeteners may not be required or may only be utilized in relatively small amounts. The optional sweetener blend is also selected in order to compliment the energy requirements of the inventive composition. To this end, in some compositions the artificial sweetener saccharin may be particularly preferred since saccharin has no calories and no nutritional value. Sucralose is a further preferred non-caloric sweetener, since this is about 600 times sweeter than the sugar sucrose.

Accordingly, digestible and/or non-digestible sweeteners may be employed in the present invention either alone or in combination. Most preferably, sweeteners are present in the inventive compositions in the ranges as indicated herein for carbohydrates.

For the purposes of the inventive composition, emulsifiers may also be present, which include, but are not limited to, mono- and diglycerides, distilled mono-glycerides, soy lecithin, egg yolk and egg lecithin, which may be present either alone or in combination.

Said optional emulsifiers may be present in an amount between about 0.05 to about 1%, preferably about 0.1 to about 0.2 %, or about 0.15 to about 0.3 %, or about 0.18 to about 0.4 %, or about 0.35 to about 0.5 %, or about 0.45 to about 0.65 %, or about 0.55 to about 0.8 %, or about 0.7 to about 0.9 % by weight of the inventive composition.

Optionally a fibre source may be employed in the inventive composition. The fibre source, if employed in the inventive composition may be either soluble or insoluble fibre which can be present alone or in combination which may be selected from the group consisting of inulin, oat fibre, gum acacia (i.e., Gum Arabic), corn fiber, wheat bran, oat bran, corn bran, whole corn flour, whole oat flour, fructooligosaccharides (from Inulin), pea fibre, partially hydrolyzed guar gum and mixtures thereof, etc. and the like either alone or in combination.

According to a preferred embodiment no fibre source is employed in the inventive composition. In an alternative embodiment, where a fibre source is present, the amount of fibre which may be present in the inventive composition typically ranges from about 0.1 to about 2.5% by weight, preferably from 0.5 to 2% by weight, preferably about 0.7 to about 1%, or about 0.8 to about 2%, or about 0.9 to about 1.5%, or about 1 to about 1.2%.

As defined before, the inventive composition typically comprises about 4 to about 12% by weight protein, less than 5% by weight carbohydrate and about 25 to about 38% by weight fat. Most preferably, the composition or ketogenic composition contains about 0 to about 23% by weight medium chain triglycerides and/or about 6 to about 35 % by weight long chain triglycerides. Furthermore, it is also particularly preferred that the inventive composition does not contain or comprise DHA, DPA,and/or ARA.

It is further preferred that 0.4 to 2.0% by weight of the fat in the inventive composition is eicosapentaenoic acid (EPA). Most preferably EPA is present in the inventive composition at about 0.6 to about 1 % by weight fat, or about 0.8 to about 1.3 %, or about 1.1 to about 1.6%, or about 1.4 to about 1.8 % by weight fat. The term "%" is preferably defined as "% by weight" and in this context reflects the total amount of the preferred EPA component

used in the inventive composition. According to an alternative, said inventive composition may contain no eicosapentaenoic acid (EPA).

For the purposes of the inventive composition, flavourings may also be present. The flavourings employed may include natural flavourings, such as essential oils e.g. lemon oil, and/or synthetic flavourings.

According to a particularly preferred embodiment, the ratio of fat: protein: carbohydrate in the inventive composition is about 4:1:0.01, wherein preferably each of the values for said ratio for fat: protein may vary by +/- 0.3. Accordingly, in some aspects ratios of 3.7: 0.7: 0.01 to 4.3: 1.3: 0.01 may be utilized. For instance, one such ratio comprised in the inventive composition is 3.8: 0.9: 0.01.

In the present invention it is additionally or alternatively preferable that the weight ratio of fat to the sum of proteins and carbohydrates in the inventive composition, is 4: 1, which may vary by +/- 0.3. For instance, preferably the weight ratio of fat to the sum of proteins and carbohydrates, preferably digestible carbohydrates is 3.7: 0.7 to 4.3: 1.3.

It is also particularly preferable that the inventive composition utilizes no artificial additives and/or utilizes whole protein. Having said this, in a preferred embodiment the composition does not utilize whole milk protein in a ratio of 80:20 casein protein to whey protein.

It is also preferable that intact proteins are comprised in the inventive composition and further preferred that casein is not present.

Furthermore, the inventive composition may also utilize linoleic acid or α-linoleic acid either alone or in combination.

Preferably, the present invention contains linoleic acid in about 1000 mg to about 4000 mg per 100g, preferably about 2000 to about 3500 mg.

Preferably, the present invention contains α-linoleic acid in about 40 to about 90 mg per 100g, preferably about 50 to about 80mg, or about 60 to about 80mg, or about 70 to about 75mg.

Acids may also be employed in the inventive composition, preferably citric acid and/or malic acid and/or ascorbic acid and/or lactic acid and/or succinic acid and/or acetic acid.

The inventive composition, which is preferably a semi-solid composition, most preferably a liquid composition is adjusted to a pH of less than about 4.3, preferably about 3.5 to about 4.2 as defined herein for the inventive composition and inventive process.

Said inventive composition may also comprise micronutrients selected from vitamins, minerals and trace elements, which may be present either alone or in combination. Alternatively, for some embodiments the inventive compositions may also not contain any micronutrients.

The term "vitamin" as used herein, refers to any of various organic substances essential in minute quantities to the nutrition of most animals act especially as coenzymes and precursors of coenzymes in the regulation of metabolic processes. Vitamins have diverse biochemical functions, including function as hormones (for example, vitamin D), antioxidants (for example, vitamin C and vitamin E), and mediators of cell signalling, regulation of cell growth, tissue growth and differentiation (for example, vitamin A). The B complex vitamins, which is the largest in number, function as precursors for enzyme cofactor bio-molecules (co-enzymes) that help act as catalysts and substrates in metabolism. For instance Vitamin B₆ and Vitamin B₁₂. Other Vitamins which may be present include Vitamin K, Thiamin, Riboflavin, Niacin, Folic Acid, Biotin and Pantothenic Acid.

The inventive composition preferably comprises the following vitamins per 100 kcal:

Vitamin A in about 35 to about 180 µg per 100 kcal, preferably at about 40 to about 80 µg, or about 70 to about 110 µg, or about 85 to about 140 µg, or about 100 to about 150 µg, or about 135 to about 170 µg per 100 kcal.

Vitamin D in about 0.5 to about 3 µg per 100 kcal, preferably at about 0.9 µg to about 1.5 µg, or about 1.3 µg to about 1.9 µg, or about 1.7 µg to about 2.2 µg, or about 2 µg to about 2.6 µg, or about 2.3 µg to about 2.8 µg per 100 kcal.

Vitamin K in about 3.5 to about 20 µg per 100 kcal, preferably at about 5 to about 9 µg, or about 7 to about 12 µg, or about 10.5 to about 15 µg, or about 13 to about 18 µg per 100 kcal.

Vitamin C in about 2.25 to about 22 mg per 100 kcal, preferably at about 5 to about 10 mg, or about 8 to about 14 mg, or about 11 to about 15 mg, or about 13 to about 18 mg per 100 kcal.

Thiamin in about 0.02 to about 0.5 mg per 100 kcal, or preferably at about 0.04 to about 0.15 mg per kcal, more preferably about 0.04 to about 0.1 mg per kcal, or about 0.1 to about 0.19 mg, or about 0.16 to about 0.23 mg, or about 0.19 to about 0.26 mg, or about 0.22 to about 0.3 mg, or about 0.28 to about 0.35 mg, or about 0.32 to about 0.4 mg, or about 0.37 to about 0.45 mg per 100 kcal.

Ribo flavin in about 0.05 to about 0.5 mg per 100 kcal, preferably at about 0.06 to about 0.19 mg per kcal, preferably about 0.06 to about 0.15 mg per kcal, or about 0.15 to about 0.23 mg, or about 0.20 to about 0.29 mg, or about 0.27 to about 0.36 mg, or about 0.33 to about 0.40 mg, or about 0.37 to about 0,45 mg per 100 kcal.

Vitamin B₆ in about 0.05 to 0.5 mg per 100 kcal, preferably at about 0.06 to about 0.19 mg per kcal, preferably about 0.06 to about 0.15 mg per kcal, or about 0.15 to about 0.24 mg, or about 0.21 to about 0.30 mg, or about 0.27 to about 0.34 mg, or about 0.30 mg to about 0.38 mg, or about 0.36 to about 0.42 mg, or about 0.39 to about 0.46 mg per 100 kcal.

Niacin in about 0.5 to 3 mg per 100 kcal, preferably at about 0.6 to about 1.9 mg, preferably about 0. 6 to about 1.5 mg per kcal, or about 1.5 to about 2.2 mg, or about 2.0 to about 2.5 mg, or about 2.2 to about 2.8 mg per 100 kcal.

Folic acid in about 10 to about 50 µg per 100 kcal, preferably at about 14 to about 20 µg, or about 18 to about 24 µg, or about 21 to about 28 µg, or about 25 to about 32 µg, or about 29 to about 34 µg, or about 31 to about 38 µg, or about 35 to about 41 µg, or about 38 to about 45 µg per 100 kcal.

Vitamin B₁₂ in about 0.07 to about 0.7 µg per 100 kcal, preferably at about 0.1 to about 0.18 µg, or about 0.14 to about 0.22 µg, or about 0.19 to about 0.25 µg, or about 0.23 to about 0.30 µg, or about 0.28 to about 0.38 µg, or about 0.35 to about 0.43 µg, or about 0.40 to about 0.48 µg, or about 0.43 to about 0.52 µg, or about 0.49 to about 0.58 µg, or about 0.54 to about 0.62 µg, or about 0.59 to about 0.65 µg, or about 0.62 to about 0.67 µg per 100 kcal.

Pantothenic acid in about 0.15 to about 1.5 mg per 100 kcal, preferably at about 0.4 to about 0.9 mg, or about 0.7 to about 1.1 mg, or about 0.9 to about 1.3 mg, or about 1.1 to about 1.4 mg per 100 kcal.

Biotin in about 0.75 to about 7.5 µg per 100 kcal, preferably at about 1.0 to about 1.9 µg, or about 1.7 to about 2.5 µg, or about 2.3 to about 3.1 µg, or about 2.8 to about 3.6 µg, or about 3.4 to about 4.2 µg, or about 3.9 to about 4.7 µg, or about 4.4 to about 5.2 µg, or about 4.9 to about 5.6 µg, or about 5.2 to about 6.3 µg, or about 5.9 to about 6.5 µg, or about 6.2 to about 7.0 µg per 100 kcal.

Vitamin E in about 0.5 to about 3 mg per 100 kcal, preferably at about 0.8 to about 1.4, or about 1.1 to about 1.8, or about 1.5 to about 2.1, or about 1.7 to about 2.5, or about 1.9 to about 2.8 mg per 100 kcal. Wherein preferably vitamin E is present in 0.5mg/g of polyunsaturated fatty acids, preferably expressed as linoleic acid but most preferably in no case less than 0.5 mg per 100 available kcal.

The term "micronutrient" as used herein refers to vitamins and minerals that are required in the human diet in very small amounts.

Dietary minerals are chemical elements other than carbon, hydrogen, nitrogen, and oxygen that are required to sustain the health of living organisms. In humans, dietary minerals can include calcium, magnesium, phosphorus, potassium, sodium, and sulphur.

The inventive composition as described herein also preferably comprises the following minerals per 100 kcal:

Sodium in about 30 to about 175 mg per 100 kcal, preferably at about 70 to about 110 mg, or alternatively about 90 to about 130 mg, or about 110 to about 150 mg, or about 135 to about 155mg, or about 140 to about 165 mg per 100 kcal.

Potassium in about 30 to about 295 mg per 100 kcal, preferably at about 50 to about 150 mg, preferably about 70 to about 100 mg, alternatively or about 125 to about 180 mg, or about 160 to about 210 mg, or about 190 to about 240 mg, or about 220 to about 270 mg per 100 kcal.

Phosphorus in about 30 to about 80 mg per 100 kcal, preferably at about 39 to about 55 mg, or about 44 to about 60 mg, or about 57 to about 65 mg, or about 61 to about 69 mg, or about 66 to about 72 mg, or about 71 to about 76 mg per 100 kcal.

Magnesium in about 7.5 to about 25 mg per 100 kcal, preferably at about 10 to about 14 mg, or about 12 to about 18 mg, or about 16 to about 20 mg, or about 19 to about 23 mg per 100 kcal.

Calcium in about 50 to about 250 mg per 100 kcal, preferably at about 90 to about 130 mg, or about 110 to about 150 mg, or about 130 to about 170 mg, or about 160 to about 190 mg, or about 180 to about 210 mg, or about 200 to about 230 mg per 100 kcal.

Likewise preferably, the composition of the present invention may comprise or contain calcium, most preferably in an amount of about 100 to about 245 mg per 100 g and/or lecithin, even more preferably in an amount of about 0.1 to about 2%.

It is more preferable that calcium is present in the present invention at about 120 to about 150 mg, or about 135 to about 190 mg, or about 185 to about 200 mg, or about 195 to about 210 mg, or about 205 to about 215 mg, or about 213 to about 220 mg per 100g.

It is particularly preferable that calcium is present at about 209 mg.

Furthermore, the inventors also found that by also employing magnesium and calcium lactate in the invention as described herein, this improved the protein stability of the product.

Preferably, calcium lactate is present in the present invention in the range of about 0.5 to about 3% by weight. More preferably, in about 0.6% to about 1.7%, or about 1.5% to about 1.8%, or about 1.6% to about 2%, or about 1.9% to about 2.5%. Particularly preferable is that calcium lactate is present in 1.76% by weight.

Preferably, magnesium lactate is present in the present invention in the range of about 0.2 to about 2% by weight. More preferably, in about 0.3 to about 0.5%, or about 0.4 to about 0.6%, or about 0.55 to about 0.8%, or about 0.7 to about 1.2%, or about 1 to about 1.5%. Particularly preferable is that magnesium lactate is present in 0.59% by weight.

The amounts of specific vitamins and minerals in the inventive composition may be determined by one of skill in the art.

Furthermore, minerals that are needed in relatively small quantities and may be referred to as trace elements, for example, chromium, cobalt, copper, chloride, fluorine, iodine, iron, manganese, molybdenum, selenium, and zinc.

The inventive composition as described herein also preferably comprises the following trace elements per 100 kcal:

Iron in about 0.5 to about 2.0 mg per 100 kcal, preferably at about 0.75 to about 1.3 mg, or about 1.1 to about 1.45 mg, or about 1.30 to about 1.65 mg, or about 1.55 to about 1.85 mg, or about 1.75 to about 1.9 mg per 100 kcal.

Zinc in about 0.3 to about 1.5 mg per 100 kcal, preferably at about 0.3 to about 1.1 mg, preferably about 0.5 to about 0.7 mg or about 0.90 to about 1.3 mg, or about 1.2 to about 1.4 mg per 100 kcal.

Copper in about 30 to about 500 µg per 100 kcal, preferably at about 40 to about 130 µg, or about 115 to about 150 µg, or about 135 to about 170 µg, or about 155 to about 200 µg, or about 180 to about 230 µg, or about 210 to about 280 µg, or about 250 to about 330 µg, or about 310 to about 390 µg, or about 375 to about 450 µg per 100 kcal.

Chloride in about 30 to about 175 mg per 100 kcal, preferably at about 70 to about 110 mg, or about 90 to about 130 mg, or about 110 to about 150 mg, or about 135 to about 165 mg, or about 155 to about 170 mg per 100 kcal.

Iodine in about 3.5 to about 35 µg per 100 kcal, preferably at about 4 to about 16 µg, such as about 5 to about 10 µg, or about 13 to about 18 µg, or about 16 to about 20 µg, or about 19 to about 25 µg, or about 22 to about 29 µg, or about 27 to about 33 µg per 100 kcal.

Selenium in about 2.5 to about 10 µg per 100 kcal, preferably at about 3.5 to about 5 µg, or about 4 to about 6 µg, or about 5.5 to about 8 µg, or about 7 to about 9 µg per 100 kcal.

Manganese in about 0.05 to about 0.5 mg per 100 kcal, preferably at about 0.1 to about 0.15 mg, or about 0.13 to about 0.19 mg, or about 0.16 to about 0.23 mg, or about 0.21 to about 0.26 mg, or about 0.22 to about 0.3 mg, or about 0.28 to about 0.35 mg, or about 0.32 to about 0.4 mg, or about 0.37 to about 0.45 mg per 100 kcal.

Chromium in about 0.5to about 15 µg per 100 kcal, preferably at about 1.0 to about 3.0 µg, or about 1.2 to about 4.3 µg, or about 4 to about 6 µg, or about 5.5 to about 7.5 µg, or about 7 to about 9 µg, or about 8.5 to about 12 µg, or about 10 to about 13 µg, or about 12 to about 14 µg per 100 kcal.

Molybdenum in about 1.0 to 18 µg per 100 kcal, preferably at about 2 to about 5 µg, or about 3 to about 6 µg, or about 5 to about 8 µg, or about 7 to about 9 µg, or about 8 to about 12 µg, or about 10 to about 13, or about 12 to about 16 µg per 100 kcal.

Fluoride in about 0 to about 0.2 mg per 100 kcal, preferably at about 0.05 to about 0.09 mg, or about 0.07 to about 0.12 mg, or about 0.10 to about 0.14 mg, or about 0.12 to about 0.18 mg per 100 kcal. According to a preferred embodiment fluoride is not employed in the inventive composition.

The inventive composition as described herein can include any combination of vitamins, minerals and trace elements that is useful in providing appropriate nutrition to the patient. The vitamins, minerals and trace elements may be used in the form of a mixture or formulation.

It is more preferable that the lecithin is present in the present invention at about 0.3 to about 0.7%, or about 0.5 to about 0.9%, or about 0.8 to about 1%, or about 0.95 to about 1.3%, or about 1.1 to about 1.5%, or about 1.4 to about 1.8%.

Particularly favourable inventive compositions may comprise or contain an antifoaming agent, preferably selected from lecithin and/or MCT oil, and/or silicone antifoamers, more preferably in an amount of about 0.5 to 23 % by weight.

Even more preferably lecithin is present in the present invention at about 0.5 to 5% by weight, preferably at about 0.6 to about 0.89%, or about 0.7 to about 1.1%, or about 1.0 to about 1.3%, or about 1.2 to about 1.6%, or about 1.4 to about 2.0%, or about 1.8 to about 2.38%, or about 2.2 to about 2.6%, or about 2.5 to about 3%.

Most preferably the present invention has an energy content per 100g of about 1397 KJ (about 325 Kcal) and preferably contains or comprises the following ingredients per 100g: protein at about 8 g, carbohydrate at about 0.1 g,
Fat at about 32.5 g;

Vitamin premix which preferably contains or comprises Vitamin A, Vitamin D, Vitamin E, Vitamin C, Vitamin K, thiamin, riboflavin, niacin, Vitamin B₆, folic acid, Vitamin B₁₂, biotin, pantothenic acid and choline. Other vitamins may also be present as defined herein above.

Minerals which preferably contains or comprises sodium, potassium, calcium, phosphorus and magnesium. Other minerals may also be present as defined herein above.

Trace element mix which preferably contains or comprises chloride, iron, copper, zinc, manganese, iodine, molybdenum, selenium and chromium. Other trace elements may also be present as defined herein above.

According to a particularly preferred aspect, the inventive composition comprises the following ingredients: Water, Vegetable Oil(High Oleic Sunflower Oil), Whey Protein Isolate , Calcium Lactate, Emulsifier(Soya Lecithin), Tri Potassium Citrate, Magnesium Lactate, Sodium Chloride, Flavouring, Choline Bitartrate, Tri Sodium Citrate, L-Ascorbic Acid, Acidity Regulator(E330), Taurine , Artificial Sweetener, L-Carnitine L-Tartrate, Zinc Gluconate, Antioxidant(E307), Ferrous Sulphate, DL-Alpha-Tocopheryl Acetate, Nicotinamide, Manganese Gluconate, Maltodextrin, Copper Gluconate, D-Calcium Pantothenate, Pyridoxine Hydrochloride, Riboflavin, Thiamin Hydrochloride, Retinyl Acetate, Folic Acid (Pteroylmonoglutamic Acid), Potassium Iodide, Sodium Molybdate, Chromium Chloride, Sodium Selenite, Vitamin K (Phylloquinone), D-Biotin, Vitamin D3 (Cholecalciferol), Vitamin B12 (Cyanocobalamin)..

The compositions as described herein are preferably either liquids or semi liquids/semi solids, although semi-solids are particularly preferred, wherein the viscosity can be tailored as desired, preferably by applying pressures as outlined herein in a process for preparing the inventive composition, which may be supplemented e.g. by adding starch as defined herein.

The term "viscosity" as used herein refers to the measure of the thickness or resistance of a fluid to flow. Liquids with a high viscosity such as semi-liquids or semi-solids are usually very thick and flow very slowly, while low viscosity liquids generally are thin and flow quickly.

The inventors have surprisingly found that for a viscous inventive composition, a homogenization pressure of about 86 to about 240 bar, preferably about 90 to about 100 bar, or about 95 to about 120 bar, or about 110 to about 140 bar, or about 130 to about 160 bar, or about 150 to about 180 bar, or about 170 to about 190, or about 190 to about 210, or about 200 to about 220 bar can be used to give a semi-liquid or semi-solid viscosity.

On the other hand, if a less viscous inventive composition is desired, lower homogenization pressures, such as about 1 to about 85 bar, preferably about 10 to about 30 bar, or about 20 to about 45 bar, or about 40 to about 65 bar, or about 55 to about 80 bar can be used in order to give a more liquid viscosity.

In order to obtain an inventive composition with a semi-solid viscosity, most preferably a homogenization pressure of about 200 bar is used.

The present inventive composition preferably encompass products as described herein having preferably liquid, semi-liquid or semi-solid viscosities defined in centipose (cP) of about 1 to about 40,000 cP, preferably about 1000 to about 40,000 cP.

Viscosities described herein were measured at 25°C using a Brookfield DV-E Viscometer (LV model). Alternatively, viscosities can also be measured by other methods known in the art. As used herein, the viscosity terms semi-solid and semi-liquid can be used interchangeably. Having said this, generally speaking, a product with a semi-solid viscosity as described herein is generally more viscous than a product with a semi-liquid viscosity.

In some aspects, for a liquid composition, the inventors have found that this is typically from about 1000 to about 4700 cP, preferably about 1100 to about 1400 cP, or about 1350 to about 1600 cP, or about 1500 to about 1750 cP, or about 1650 to about 2000 cP, or about 1900 to about 2300 cP, or about 2100 to about 2600 cP, or about 2500 to about 2900 cP, or about 2800 to about 3100 cP, or about 3000 to about 3200 cP, or about 3150 to about 3500 cP, or about 3400 to about 3900 cP, or about 3800 to about 4200 cP, or about 4100 to about 4600 cP.

In a further aspect of the inventive composition, without being limited thereto, a liquid can also be further described as being honey-like which is about 351 to about 1750 cP.

As used herein, with regard to viscosity the term "honey-like" refers to liquids that have been thickened to honey consistency. The liquid flows off a spoon in a ribbon, just like actual honey.

The inventors have also surprising found that for the inventive composition a semi-liquid typically has a viscosity from about 4500 cP to about 25,000 cP, preferably about 4600 to about 5000 cP, or about 4800 to about 5400 cP, or about 5200 to about 5800 cP, or about 5700 to about 6500 cP, or about 6200 to about 7000 cP, or about 6900 to about 10,000 cP, or about 9,000 to about 12,000 cP,.

The inventors have also surprising found that for the inventive composition a semi-solid typically has a viscosity from about 12,000 to about 40,000 cP, preferably about 12,000 to about 16,000 cP, or about 14,000 to about 16,000 cP or about 14,000 to about 20,000 cP, or about 19,000 to about 22,000 cP about 19,000 to about 22,000 cP, or about 21,000 to about 25,000 cP, or about 24,000 to about 30,000 cP, or about 26,000 to about 32,000 cP, or about 31,000 to about 35,000 cP, or about 34, 000 to about 37,000 cP, or about 36,000 to about 39,000 cP.

Furthermore, as defined for the inventive process herein, the viscosity of the inventive composition can also preferably be selected depending on the processing conditions utilized such as homogenization pressure, single or multistage homogenization (such as first stage followed by a second stage), holding time, pasteurization temperature, the presence or exclusion of starch, amount of protein employed and the type of sealable packaging used etc. Wherein said processing conditions can be selected either alone or in combination.

As used herein, the abovementioned descriptive and/or measured viscosities correspond to the viscosities as determined at the start of the shelf-life as defined herein for the inventive process.

The inventive composition, which is preferably a semi liquid/semi solid composition, most preferably a semi-solid composition, may be adjusted to a pH of less than about 4.3, preferably to a range of about 3.5 to about 4.5. Most preferably the pH of the inventive composition is in such a range, more preferably about 4.3 or, even more preferably about 4.2.

It is particularly preferable that the inventive composition provides an energy content of about 300 to about 380 kcal, preferably about 300 to about 360 kcal, most preferably, about 325 Kcal per 100g, preferably per unit or dose for administration. Such a unit or dose for administration may be a bottle, pot, tear top tube or a pouch, which is preferably a Guala pack etc.

Preferably, such a unit or dose for administration exhibits a weight of about 30 to 200 g of the inventive composition, more preferably a weight of about 30 to 150 g, even more preferably a weight of about 70 to 110 g. Most preferably such a unit or dose for administration comprises e.g. a 30g, 50g pouch, a 70g pouch, a 80g pouch, a 90 g pouch, a 100 g pouch, a 150 g pouch, a 200 g pouch, etc., or any further unit or dose for administration or as defined herein. The unit or dose for administration as described herein can also be expressed in terms of mL rather than g. Hence, if desired 30 to 200 mL of the inventive composition can be employed. The total amount of energy to be administered by the inventive composition per day is preferably in range of about 300 to about 635 kcal, most preferably about 300 to about 600 kcal.

According to a particularly preferable aspect, the inventive composition may be in form of a supplement (and may be packaged accordingly), preferably providing a unit or dose for administration as defined before, e.g. an amount as described above, e.g. an amount of about 1 to about 2 x 100 g pouches a day or for instance of about 1 to about 2 x 50 g pouches a day. Preferably the total amount of energy to be administered per day is between about 300 to about 635 Kcal, most preferably about 300 to about 600 Kcal a day, preferably if used as a supplement.

Alternatively, the inventive composition may preferably be used and packaged as a sole source of nutrition (e.g. as a whole meal), preferably providing a unit or dose for administration as defined before, e.g. an amount as described above, e.g. an amount of about 4 to about 8 x 100g pouches a day or for instance of about 4 to about 8 x 50g pouches a day. Preferably the total amount of energy administered using the inventive composition per day is typically in the range of about 1200 to about 2500 Kcals a day, preferably if used as a sole source of nutrition (e.g. as a whole meal).

Further inventive compositions as detailed herein are also suitable for use in dietary modification or manipulation involving the intake of fat, protein and carbohydrate, in isolation or combination, for therapeutic effect and benefit.

A further advantage of the inventive composition comprising less than 5% carbohydrate is that in addition to the inventive composition additional foods, or protein or carbohydrate sources can be added in prescribed amounts to a diet whilst maintaining an effective ketogenic fat to protein to carbohydrate ratio. This permits patients an increased degree of flexibility in their diet, which is beneficial amongst others for facilitating patient compliance.

Preferably, the composition of the present invention, which is most preferably a ketogenic composition, could be obtained by any process suitable for a skilled person. More preferably, the inventive composition may be obtained or is obtainable by a method for preparing a composition as defined herein. Such a composition and any composition obtained or obtainable by such a method for preparing a composition may be used for treatment of a disease or disorder as defined herein, particularly preferably for use in treating epilepsy, preferably refractory epilepsy in children. It is most preferred that such a composition is for use in treating disorders treatable with the ketogenic diet and selected from neurodegenerative diseases, and improvement of brain function or of cognitive skills, Alzheimer disease/cognitive impairment, Parkinson's disease, neurological diseases, Amyotrophic lateral sclerosis, Traumatic brain injury, Hypoxic/ischemic brain injury, Autism, ADHD (Attention Deficit Hyperactivity Disorder), Depression, Headaches, Migraine Headaches, Narcolepsy, GLUT-1 deficiency, Pyruvate Dehydrogenase (PDH) deficiency, phosphofructokinase (PFK) deficiency, Glycogenosis type V (McArdle disease), Cardiac ischemia, Rett syndrome, Tuberous Sclerosis, Diabetes and Cancer (astrocytornas, prostate, gastric, renal, head and neck).

According to a further embodiment, the object underlying the present invention is preferably also solved by a process for preparing a composition, preferably a composition as defined herein, more preferably a ketogenic composition as described herein. The present invention hence describes a composition as described above, preferably a composition obtained or obtainable according to a process for preparing such a composition as defined herein. In this regard, said process may contain or comprise any of the amounts and ingredients as defined for the inventive composition.

Hence, according to a particularly preferred aspect the object underlying the present invention is solved by a process for the preparation of a composition, preferably a ketogenic composition, more preferably a composition as described herein, comprising the following steps:
(a) Mixing about 4 to about 12% by weight protein, optionally carbohydrate in an amount to less than 5% by weight carbohydrate and about 25 to about 38% by weight fat with water;
(b) Optionally preheating the mixture;
(c) Homogenizing the mixture obtained according to step (a) or (b) at a temperature of about 86 to about 92°C, preferably at a pressure of about 30 to about 200bar.
(d) Packaging the mixture into a package, which is, preferably a sealable package, which is preferably a pouch, bottle, tear top tube or pot, which is preferably a unit or dose suitable for administration;
(e) Pasteurizing the packaged mixture at a temperature of about 73°C, preferably about 76°C to about 93°C, preferably at a time of about 2 to about 10 minutes;
(f) Preferably cooling the pasteurized packaged mixture.

In this regard, any of the steps, in particular, steps (a) to (c) can be repeated as necessary - either independently or in combination.

According to a first step (a) of the inventive process about 4 to about 12% by weight protein, less than 5% by weight carbohydrate and about 25 to about 38% by weight fat are mixed with water. Such a mixture preferably comprises proteins, fats and/or carbohydrates as defined herein, preferably as defined herein for the inventive composition, more preferably in the preferred ranges as defined herein. Most preferably, ingredients including those as defined herein are added at step (a). In other words, it is most preferred that no further ingredients are added in steps (b) to (f).In case further ingredients are to be added at a later stage, preferably steps (a), (b) and/or (c), and optionally further steps as defined herein may be repeated at least once, allowing further addition of ingredients as defined herein, preferably in the preferred ranges as defined herein, preferably during such a repeated step (a).

Preferably, after step (c) the homogenized product is sent to a filler head prior to packing step (d).

Step (c) can also be viewed as a first stage pasteurization, since said step also involves heat treatment of the mixture from step (a) or (b) using pasteurization conditions. Accordingly, step (e) which is carried out on the packaged mixture can also be seen as a second stage pasteurization step. Having said this, since step (e) is preferably followed by cooling step (f), step (e) is deemed the main pasteurizing step of the inventive process.

As used herein, pasteurization is a heat treatment step carried out at between about 73 to about 93°C, preferably at a time of about 2 to about 10 minutes, preferably followed by cooling immediately.

Most preferably pasteurization is carried out as described herein. As used herein, pasteurization has the effect of inhibiting microbial growth in the inventive compositions and process thereof. Notably, unlike sterilization, pasteurization does not kill all microorganisms in the composition, but instead reduces the number of viable pathogens and hence greatly reducing the likelihood of any pathogens present causing disease.

However, for some compositions it may alternatively be preferable to carry out a sterilization step instead of the most preferred pasteurization step. For such purposes, high temperatures are used, preferably at least above about 93°C, more preferably above about 94°C. Sterilization may preferably also be carried out at high temperatures of about 94 to about 140°C, more preferably at about 95 to about 105°C, or about 98 to about 110°C, or about 105 to about 120°C, or about 110 to about 130°C, or about 120 to about 135°C.

For the purposes of the present invention, sterilization can only potentially be achieved by heat sterilization, wherein high temperatures are employed as described herein above. Likewise preferably, the time and pressures used for sterilization are as defined herein for pasteurization.

Furthermore, other methods known in the art for achieving sterilization such as using chemical or radiation for instance do not form part of the present invention.

According to step (d), it is particularly preferable that said packaging step also involves sealing said packaged mixture prior to pasteurization step (e), wherein preferably sealing is achieved by capping the packaged mixture.

Furthermore, it is particularly preferable that the package or sealable package is a bottle, pot, tear top tube or a pouch, wherein said pouch is most preferably a Guala pack and the like. It is also preferable that the package or the sealable package is a tetra pack.

The product of the present invention is preferably further treated to avoid browning by methods known in the art by the skilled person, including purging with an inert gas such as nitrogen and/or CO₂ either alone or in combination and/or the process being vacuum sealed. Said treatment can be performed at the end of the inventive process or directly before, during or after any step or steps (a) to (f) throughout said inventive process. Preferably, such treatment is carried out in step (d).

It is particularly preferable that if degassing/vacuuming is undertaken that this occurs at step (a), and/or before step (b) of the inventive process. Furthermore, it is also preferable that any purging with inert gas occurs at step (d) of the inventive process.

Even more preferably the mixture is a composition as defined herein for the inventive composition. In a desirable aspect of the process of the present invention, the protein, the fat and/or the carbohydrate is/are derived from at least two different sources.

The mixture formed or obtained according to step (a) of the process of the present invention may contain or comprise any of the ingredients defined above for the inventive composition, preferably in the defined amounts and ratios as defined herein.

It is also particularly preferable that the inventive process utilizes no artificial additives and/or utilizes whole protein. Having said this, in a preferred embodiment the process does not utilize whole milk protein in a ratio of 80:20 casein protein to whey protein.

It is also preferable that intact proteins are used in the inventive process and further preferred that casein is not used in said process.

It is particularly preferable that in the process of the present invention, said mixture formed or obtained according to step (a) does not comprise soy protein and/or does not comprise olive oil or canula oil.

Furthermore, it may also particularly preferable that in the process of the present invention, said mixture formed or obtained according to step (a) does not contain or comprise DHA, DPA and/or ARA.

It is particularly preferable that in the process of the present invention, said mixture formed or obtained according to step (a) that 0.4 to 2.0% by weight of the fat is eicosapentaenoic acid (EPA). According to an alternative, such inventive process may contain no eicosapentaenoic acid (EPA).

It is also preferable that in the process of the present invention, said mixture formed or obtained according to step (a) contains emulsifiers and/or fibre as defined herein above for the inventive composition.

Furthermore, the inventive process may also utilize linoleic acid or α-linoleic acid either alone or in combination as defined for the inventive composition.

Preferably, in the process of the present invention the mixture of step (a) is adjusted to a pH of less than about 4.3, preferably about 3.5 to about 4.5, more preferably to a pH of about 3.7 to about 4.1 or about pH 3.8 to about 4.3, preferably about pH 4.2. In the present invention it is preferred that the pH adjustment as described herein is achieved using citric acid and/or malic acid and/or lactic acid and/or succinic acid and/or acetic acid or the like, and/or mixtures thereof. Acids can be added in liquid or dry form such as in hydrated or anhydrous form and the like". Compositions obtained by the present invention may consequently also exhibit a pH as defined before.

According to a further preferred aspect the product obtained or the mixture prepared or obtained according to step (a) of the process of the present invention additionally contains zero to about 23% by weight medium chain triglycerides and/or about 6 to about 35 % by weight long chain triglycerides as already defined for the inventive composition.

It is likewise highly desirable that the mixture formed or obtained according to step (a) of the process of the present invention contains or comprises fat at about 2 to about 30% by weight saturates, about 2 to about 30% by weight mono-unsaturates and about 0.1 to about 5% by weight poly-unsaturates. These percentages are preferably defined with regard to the entire amount of the mixture.

According to a particularly preferred aspect the protein employed in step (a) of the inventive process is as defined herein for the inventive composition, preferably a whey protein, more preferably a whey protein isolate and even more preferably an acidified whey protein isolate.

It is particularly preferable that in step (a) of the inventive process the mixture contains an antifoaming agent as defined above, preferably selected from lecithin and/or MCT oil, and/or silicone antifoamers, more preferably in an amount of about 0.5 to about 23 % by weight, most preferably in amounts of about 1 to about 5 % by weight or as defined earlier. For the present invention MCT oil not only acts as an anti-foaming agent, but is also nutritionally required.

In this regard, the inventors found that in the absence of said antifoaming agents, whey protein or the like present may be prone to being aerated and during the mixing phase this may result in excessive foaming which can lead to processing difficulties in terms of pumping and homogenising the product.

The fat source used in the inventive process can be a 100% LCT or 100% MCT fat source, which can be employed either alone or in combination. Accordingly, the inventive process preferably may provide compositions containing a mixture of MCT and LCT as defined herein. Alternatively, if for instance solely a MCT fat source is utilized, this provides compositions which may lack an LCT component. If for instance solely a LCT fat source is utilized, this provides compositions which may lack an MCT component. In one preferred embodiment the fat source used is a 100% LCT fat source.

Said mixture in step (a) and compositions prepared by the present process also preferably comprise micronutrients selected from vitamins, minerals and trace elements, which may be present either alone or in combination as defined for the inventive composition herein.

The terms and ranges for the vitamins, minerals and trace elements and the like as applicable for the inventive process have already been described herein for the inventive composition.

Said mixture in step (a) also preferably comprises calcium as defined before, most preferably in an amount of about 100 to about 245 mg per 100 g.

Furthermore, the inventors also found that by preferably using magnesium and calcium lactate in the mixture formed or obtained according to step (a) of the process of the present invention, the protein stability of the product may be significantly improved.

Further to the above, mixing is carried out in step (a) of the inventive process. For the inventive process it is specifically preferred that during step (a) mixing speeds are employed, which typically range from about 320 rpm to about 800 rpm, preferably about 377 to about 725 rpm, or about 360 to about 700 rpm, or about 370 to about 750 rpm, or about 374 to about 740 rpm, or about 380 to about 730 rpm. For some of the ingredients it is preferable to employ slow mixing speeds which are preferably of about 320 to about 400 rpm, preferably about 360 to about 380 rpm, or about 370 to about 390 rpm.

Furthermore, for some ingredients it may be preferable to employ medium mixing speeds of preferably about 401 to about 550 rpm, or about 410 to about 430 rpm, or about 420 to about 450 rpm, or about 440 to about 490 rpm, or about 460 to about 500 rpm.

Furthermore, for some ingredients it may be preferable to employ medium-fast mixing speeds, preferably of about 551 to about 800 rpm, or about 510 to about 550 rpm, or about 530 to about 580 rpm, or about 560 to about 640 rpm, or about 600 to about 700 rpm, or about 660 to about 750 rpm.

Even more preferably, stepwise mixing speeds of about 725 rpm followed by about 377 rpm or about 435 rpm followed by about 435 rpm followed by about 580 rpm, followed by about 435 rpm, followed by about 580 rpm, followed by about 725 rpm or the like are employed. Furthermore, combinations around stepwise mixing speeds using the aforesaid slow, medium and medium-high ranges may also be employed.

Following the above mentioned mixing steps the pH is then checked to ensure that this is less than 4.3, preferably in the range from about pH 3.5 to about pH4.2, and if necessary said mixture is adjusted with citric acid to obtain the desired pH.

In this regard, the inventors found that for some of the ingredients, if slow mixing was not employed during step (a) of the process of the present invention this may result in excessive aeration, which in turn may lead to problems in the homogenization step, namely a highly aerated product may not run through the homogenizer.

It is highly preferable that for the process of the present invention that mixing employed during step (a) of the process of the present invention is conducted with a twin impeller agitator and/or using a Jex mixing tank. Alternatively, any low sheer/low agitation mixing tank (with variable speed agitation) would be suitable.

It is most preferred that if a Jex mixing tank is used in the process as described herein that this preferably has the following dimensions:
Internal diameter of about 1500 mm, top of top cone to bottom of bottom cone of about 1800 mm, height of cylindrical section of about 1250 mm, bottom mixer from sloping base of about 230 mm, bottom mixer diameter of about 180 mm with 4 blades, top mixer to bottom mixer of about 300 mm, top mixer diameter of about 300 mm with 3 blades.

It is particularly preferable in the process of the present invention that the mixing step as described for step (a) of the process of the present invention is carried out at about 10 to about 15°C, or about 13 to about 19°C, or about 18 to about 26°C, or about 25 to about 32°C, or about 30 to about 36°C, or about 34 to about 43°C, or about 15 to about 25°C, or about 12 to about 20°C, or about 18 to about 30°C or more preferably at about 17 to about 23°C.

The aforementioned temperature ranges can also be used for any preheating if carried out.

It is further preferred in the inventive process as described herein that during step (a) the order of reagent addition is preferably water, lecithin, protein - which is preferably acidified whey protein, sunflower oil, most preferably high oleic sunflower oil, vitamins, minerals, and trace elements, antioxidants vitamin E and C, sweeteners and flavours. Further components may be added as defined for the inventive process or the inventive compositions as described herein. It is preferable that the pH is then checked to ensure that this is less than about 4.3.

Wherein during the course of the reagent addition the temperature is preferably kept in one of the temperature ranges as listed above for mixing step (a), although it is more preferred that several of the abovementioned temperature ranges are employed for mixing step (a) during reagent addition. For instance, 34 to about 40°C, or 35 to about 43°C for the mixing in of water, lecithin and starch, followed by about 37 to about 43°C, most preferably at about 35 to about 40°C for instance for the mixing in of acidified whey protein.

The inventors have also surprisingly found that if the protein employed in the mixture according to step (a) of the inventive process is acidified whey protein this leads to an improved viscosity and taste compared to when standard whey protein isolates are employed.

As described herein, the inventive process is most preferably carried out as a continuous process although said inventive process can also preferably be carried out as a batch process.

Following a particularly preferable protocol for step (a) of the inventive process as described herein, water may be added to a mixing tank, which is preferably a Jex mixing tank or the like, preferably at 20°C +/- 3°C , wherein mixing is typically conducted with a twin impeller agitator. Preferably lecithin is then added to the tank, and the mixer speed is preferably set at about 725 rpm, most preferably mixing for about 5 minutes. Preferably a protein, most preferably acidified whey protein is then added, and the mixer speed pref-erably reduced to about 435 rpm, followed preferably by further mixing for about 30 minutes. Preferably high oleic sunflower oil is then added, followed by preferably increasing the mixer speed to about 580rpm for about 5 minutes. After said mixing it is also preferable that the mixer speed is reduced to about 435 rpm, and most preferably mixed for a further 30 minutes. Preferably then any further ingredients such as vitamins, minerals and trace elements are then added, wherein preferably the mixer speed increases to about 580 rpm, preferably mixing for about 10 minutes. It is preferable that the antioxidants such as vitamin C and vitamin E, sweeteners and flavours are then added, followed by mixing for about 10 minutes at about 725rpm The pH is then checked to ensure that this is less than about 4.3, preferably in the range of about 3.5 to about 4.2, and then preferably adjust as is necessary with citric acid to fall within said limits. Preferably the product is left to degas at a mixing speed of about 725 rpm or higher for about 12 minutes, wherein optionally the product is processed to a Specific Gravity of about 1.01 at 20°C +/- 3°C or of about 1.05 at 20°C +/- 3°C.

According to step (b) of the inventive process, the mixture obtained according to step (a) is optionally preheated. Preheating may be carried out at a temperature range of about 10 to about 35°C, or about 15 to about 25°C, or about 12 to about 20°C, or of about 18 to about 30°C or more preferably at a temperature range of about 17 to about 23°C.

As furthermore described herein for step (c) of the inventive process, preferably the mixture obtained according to step (a) or (b) is homogenized at a temperature range of about 86 to about 92°C, preferably at a temperature range of about 80 to about 88°C, or about 77 to about 91°C, or about 84 to about 92°C, or about 86 to 89°C, or about 87 to about 91°C, or about 90 to about 94°C.

Homogenizing is preferably is carried out at pressures of about 15 to about 220 bar, pref-erably at about 20 to about 185 bar, or about 30 to about 170 bar, or about 40 to about 150 bar, or about 50 to about 135 bar, or about 80 to about 120 bar, or about 100 and about 140 bar, or about 18 to 22 bar, or about 25 to about 45 bar, or about 40 to about 65 bar, or about 50 to about 70 bar, or about 60 to about 75 bar, or about 90 to about 115 bar, or about 110 to about 120, or about 190 to about 210, or about 200 to about 220 bar.

Said preferable homogenization step employs homogenizers known in the art, particularly preferable homogenizers include Tetra Alex S 15, APV or Nitro Homogenizers.

For the purpose of step (c) of the inventive process, the mixture is optionally passed through an inline sieve. This (sub-) step is preferably followed by heating the mixture to about 86 to about 92°C, preferably for a minimum of about 2 minutes. The heated mixture is then typically homogenized using a homogenizer as known to a skilled person applying the defined temperatures and pressures.

The inventors have also surprisingly found that by adjusting the homogenization pressure that the viscosity of the product can be controlled. For terms regarding viscosity we refer back to the inventive composition as described herein above.

For instance, if a viscous product is desired, a homogenization pressure of about 86 to about 220 bar, preferably about 90 to about 100 bar, or about 95 to about 120 bar, or about 110 to about 140 bar, or about 130 to about 160 bar, or about 150 to about 180 bar, or about 170 to about 190 bar, or about 190 to about 210, or about 200 to about 220 can be used to give a semi-liquid or semi-solid viscosity. In one preferred embodiment a homogenization pressure of about 190 to about 210 bar is used.

On the other hand, if a less viscous product is desired, lower homogenization pressures such as about 1 to about 85 bar, preferably about 10 to about 30 bar, or about 20 to about 45 bar, or about 40 to about 65 bar, or about 55 to about 80 bar can be used in order to give a more liquid viscosity.

In order to obtain a liquid viscosity, most preferably a homogenization pressure of about 70 bar is used.

The present inventive process and composition thereof preferably encompass products as described herein having liquid, semi-liquid or semi-solid viscosities defined in centipose (cP) of about 1 to about 40,000 cP, preferably about 1000 to about 40,000 cP.

Viscosities described herein were measured at 25°C using a Brookfield DV-E Viscometer (LV model). Alternatively, viscosities can also be measured by other methods known in the art. As used herein, the viscosity terms semi-solid and semi-liquid can be used interchangeably. Having said this, generally speaking, a product with a semi-solid viscosity as described herein is generally more viscous than a product with a semi-liquid viscosity.

In some aspects, for a liquid product, the inventors have found that this is typically from about 1000 to about 4700 cP, preferably about 1100 to about 1400 cP, or about 1350 to about 1600 cP, or about 1500 to about 1750 cP, or about 1650 to about 2000 cP, or about 1900 to about 2300 cP, or about 2100 to about 2600 cP, or about 2500 to about 2900 cP, or about 2800 to about 3100 cP, or about 3000 to about 3200 cP, or about 3150 to about 3500 cP, or about 3400 to about 3900 cP, or about 3800 to about 4200 cP, or about 4100 to about 4600 cP.

In a further aspect of the inventive process and product thereof, without being limited thereto, a liquid can also be further described as being honey-like which is about 351 to about 1750 cP as already described for the inventive composition.

The inventors have also surprising found that for the inventive process and product thereof a semi-liquid is typically from about 4500 cP to about 25,000 cP, preferably about 4600 to about 5000 cP, or about 4800 to about 5400 cP, or about 5200 to about 5800 cP, or about 5700 to about 6500 cP, or about 6200 to about 7000 cP, or about 6900 to about 10,000 cP, , or about 9,000 to about 12,000 cP.

The inventors have also surprising found that for the inventive composition a semi-solid typically has a viscosity from about 12,000 to about 40,000 cP, preferably about 12,000 to about 16,000 cP, or about 14,000 to about 16,000 cP or about 14,000 to about 20,000 cP, or about 19,000 to about 22,000 cP about 19,000 to about 22,000 cP,.

The inventors have also surprising found that for the inventive process and product thereof a semi-solid is typically from about about 12,000 to about 40,000 cP, preferably about 12,000 to about 16,000 cP, or about 14,000 to about 16,000 cP or about 14,000 to about 20,000 cP, or about 19,000 to about 22,000 cP cP, or about 21,000 to about 25,000 cP, or about 24,000 to about 30,000 cP, or about 26,000 to about 32,000 cP, or about 31,000 to about 35,000 cP, or about 34, 000 to about 37,000 cP, or about 36,000 to about 39,000 cP.

The viscosities as described herein can apply for the product after step (c) of the inventive process and/or the product after step (f) of the inventive process. Depending on the processing conditions selected as described herein, such as homogenization pressure, pasteurization temperature and the type of sealable packaging used etc., the viscosity at step (c) may differ from that of step (f).

As used herein, the abovementioned descriptive or measured viscosities correspond to the viscosities as determined at the start of the shelf-life, which preferably commences after either the final process step, more preferably final process step (e). The final step of the process of course preferably depending on whether all aforementioned preceding steps are performed. Preferably, the viscosity is determined within about one minute to about eight hours of said final process step, preferably one minute to about four hours, more preferably one hour to about four hours, most preferably after about two to about four hours, or about three to about six hours, or about five to about seven hours of the final process step. The viscosity may likewise be determined subsequent to the optional cooling step (f), preferably in the aforementioned time ranges.

More specifically, the inventive process as described herein can be used to produce compositions which are preferably either liquids or semi liquids/semi solids, wherein the viscosity can be tailored as desired, preferably by applying pressures as outlined above in a process for preparing a composition, which may be supplemented e.g. by adding starch as defined herein.

In this regard, the inventors have also surprisingly found that when step (c) of the inventive process is repeated this can yield a product having an increased viscosity than if step (c) were only conducted once. In some cases, further repetitions of said step may also be desirable. For instance, step (c) could first be carried out with the homogenizer set at 70 bar pressure, denoted herein as first stage homogenization. This may then be followed by repeating step (c), denoted herein as second stage homogenization, wherein for instance the homogenizer is set at about 18 bar pressure or at a pressure as defined herein. In some cases, even a third or fourth stage homogenization may also be desirable. Preferably during second stage homogenization lower pressures are used than in the first stage homogenization. Furthermore, if a third stage homogenization is employed this is preferably at lower pressures than in the second stage homogenization.

Surprisingly, in some cases, even if the second stage homogenization employs a lower pressure compared to after the first stage homogenization step, this can lead to a two-fold, preferably a threefold increase in viscosity. Accordingly, when highly viscous products are desired, preferably the inventive process as described herein includes at least a first and second stage homogenization step.

Having said, this, when starch is employed in the inventive process as described herein, conducting a second stage homogenization step rather tends to result in either effectively no change in the viscosity, or even a reduction in viscosity compared to after the first stage homogenization step. Accordingly, if a highly viscous product is desired, if starch is employed, preferably the second stage homogenization step is omitted. In other words, when a highly viscous product is desired, when for instance a first and second stage homogenization step is conducted preferably starch is not used therein.

Alternatively, for a product with a viscosity in the lower range as disclosed herein, the inventive process preferably also includes starch along with a second stage homogenization step. Furthermore, preferably by employing protein at the higher range as described herein also tends to lead to products with a higher viscosity.

Most preferably, the inventive process herein and/or the product thereof of said process has a water content of about 40 % to about 65 % by weight, more preferably about 41 to about 43 %, or about 42 to about 45 %, or about 43 to about 46 %, or about 45.5 to about 47.5 %, or about 46.5 to about 49 %, or about 48 to about 52 %, or about 50 to about 55 %, or about 53 to about 59 %, or about 57 to about 61 %, or about 60 to about 63.5 % by weight. It is most preferred that the inventive product herein and/or the product of said process has a water content of about 53 % by weight.

According to a further optional step the product prior to the homogenization step and obtained according to step (a) or (b) is processed at Specific Gravity 1.01 at 20°C +/- 3°C or of about 1.05 at 20°C +/- 3°C prior to carrying out homogenization in step (c).

According to step (d) of the inventive process, the mixture, typically the homogenized mixture obtained according to step (c), is preferably packed into a package, which is preferably a sealable package which is preferably a unit or dose for administration as described herein, more preferably into a pot, bottle, tear top tube or into a pouch such as a Guala pack.

A pouch is particularly preferred since this advantageously allows to pasteurize the mixture in the unit or dose for administration without the risk of damaging the package. It is also preferred that the pouch is most preferably a cheerpack, pillow bag, tear-top pouch, or stand-up pouch, which is most preferably a Guala pack.

Indeed, the inventors have also found that for the present invention the pouch is particularly preferable since this is complementary to the viscosity of the inventive compositions. In this regard, if any other type of pack is used the product may thicken too much to be consumed directly from the pack. More specifically, since thickening of the inventive composition occurs inside said packaging this allows for higher protein addition and hence if desired a more viscous semi-solid inventive composition can be produced.

As furthermore described herein for step (d) of the inventive process, the homogenized product is then preferably packed directly into a package, which is preferably a sealable package, preferably the unit or dose for administration as described herein, particularly a pouch, pot, or bottle. Most preferably the pouch is a Guala pack or the like.

In step (e) of the inventive process as described herein pasteurizing the packaged mixture is carried out preferably at a temperature of about 60 to about 96°C, preferably at a time of about 2 to about 10 minutes.

It is particularly preferable that in the inventive process the pasteurization temperature is about 60 to about 96°C, preferably about 73 to about 93°C or about 65 to about 80°C, or about 70 to about 85°C, or about 76 to about 90°C, or about 82 to about 94°C.

It is particularly preferable that in the inventive process the pasteurization time is of about 2.5 to about 3.5 minutes, or about 3 to about 4.5 minutes, or about 4 to about 6 minutes, or about 5 to about 8 minutes.

Furthermore, it is particularly preferable to utilise pasteurization incorporating a scrape surface heat exchanger to ensure a build up of product is avoided, hence reducing the atherosclerotic effect created during in particular UHT processing.

Most preferably, pasteurization as described in the process herein is conducted at about 80°C to about 95°C, preferably at about 80°C to about 90°C, or at about 85°C to about 93°C, preferably about 90-93°C, or at about 87°C to about 91°C, for about 2 minutes, in the scrape surface heat exchanger and then held at about 73°C to about 93°C, or at about 73°C to about 79°C, or at about 76°C to about 80°C, or at about 77 to about 93°C, or at about 78°C to about 82°C, preferably 80°C for about 3 minutes, preferably less than 5 minutes.

For step (e) of the inventive process as described herein, pasteurizing is preferably followed by carrying out pasteurization into a package, preferably a sealable package, preferably as a unit or dose for administration as described herein, particularly preferable is a pot, bottle, tear top tube or a pouch, wherein said pouch is most preferably a Guala pack or the like, typically at conditions as described above, e.g. at about 76 to about 93°C for 3 minutes +/- 30 seconds.

When a highly viscous product is desired, optionally a first and second stage homogenization step is conducted by repeating step (c) of the inventive process herein. In one preferred embodiment in the second omogenisation step lower homogenization pressures such as about 1 to about 85 bar, preferably about 10 to about 30 bar, or about 20 to about 45 bar, or about 40 to about 65 bar, or about 55 to about 80 bar may be used

The inventors have also surprisingly found that in the present invention if the protein employed is acidified whey protein this leads to an improved viscosity and taste compared to when standard whey protein isolates are employed.

Furthermore, the inventors also found that by also employing magnesium and calcium lactate in the invention as described herein, this improved the protein stability of the product.

Preferably, calcium lactate is present in the present invention in the range of about 0.5 to about 3% by weight. More preferably, in about 0.6% to about 1.7%, or about 1.5% to about 1.8%, or about 1.6% to about 2%, or about 1.9% to about 2.5%. Particularly preferable is that calcium lactate is present in 1.76% by weight.

Preferably, magnesium lactate is present in the present invention in the range of about 0.2 to about 2% by weight. More preferably, in about 0.3 to about 0.5%, or about 0.4 to about 0.6%, or about 0.55 to about 0.8%, or about 0.7 to about 1.2%, or about 1 to about 1.5%. Particularly preferable is that magnesium lactate is present in 0.59% by weight.

Finally, as described herein for step (f) of the inventive process, this is then preferably followed by cooling in a cooling bath in order to obtain a product which is preferably less than 30°C at the end of cooling. Most preferably, said cooling step is conducted at between about 0 and about 10°C, preferably about 5 to about 15°C, or about 12 to about 20°C, or about 18 to about 25°C, or about 22 to about 29°C. Preferably the product is then packed into boxes.

Preferably, said cooling step (f) is conducted over less than about 10 minutes, or over less than about 5 minutes or over less than about 2 minutes. Most preferably said cooling step is carried out over about 1 to about 5 minutes, or about 3 to about 8 minutes or about 6 to about 10 minutes.

The inventive process as described herein has a flow rate which depends on the sealable package size along with the filling rate. For instance, when a 100g sealable package, preferably a pouch is employed as described herein, it is preferable that when using one filler about 85 pouches per minute are filled, which amounts to a flow rate of about 510 litres per hour (lph). Wherein said filler or fillers is/are placed directly before said sealable package.

Accordingly, if the inventive process employs for instance two fillers the flow rate doubles and hence increases to about 1020 lph.

Furthermore, the inventive process may also have a flow rate of about 250 to about 3500 lph, preferably about 350 to about 600 lph, or about 450 to about 700 lph, or about 650 to about 850 lph, or about 750 to about 950 lph, or about 800 to about 1300 lph, or about 1200 to about 1600 lph, or about 1400 to about 1800 lph, or about 1700 to about 2100 lph, or about 1900 to about 2500 lph, or about 2300 to about 3000 lph, or about 2800 to about 3250 lph.

It is most preferred that in the process according to the present invention the pasteurized packaged mixture is a liquid or a semi liquid/semi solid, particularly preferable is a semi-solid.

Most preferably still is that the process of the present invention, particularly the compositions produced thereof have a ratio of fat to the sum of proteins and carbohydrates of about 4:1, wherein preferably each of the values for the ratio of fat: the sum of proteins and carbohydrate may vary by +/- 0.3 as defined above.

It is also desirable that in the process of the present invention the energy content of the pasteurized packaged mixture is about 300 to about 380 kcal, preferably about 300 to about 360 kcal per 100g. Said pasteurized packaged mixture may then be administered once daily, preferably twice daily, more preferably three times daily, wherein each dose preferably comprises a 30g, 50g pouch, most preferably a 100g pouch.

It is also highly preferable that in the process of the present invention for said pasteurized packaged mixture the total amount of energy contained and suitable to be administered per day is about 300 to about 635 kcal, preferably about 300 to about 600 kcal.

According to a particularly preferred aspect, the inventive process leads to a composition as defined before, preferably comprising as ingredients Water, Vegetable Oil(High Oleic Sunflower Oil), Whey Protein Isolate , Calcium Lactate, Emulsifier(Soya Lecithin), Tri Potassium Citrate, Magnesium Lactate, Sodium Chloride, Flavouring, Choline Bitartrate, Tri Sodium Citrate, L-Ascorbic Acid, Acidity Regulator(E330), Taurine , Artificial Sweetener, L-Carnitine L-Tartrate, Zinc Gluconate, Antioxidant(E307), Ferrous Sulphate, DL-Alpha-Tocopheryl Acetate, Nicotinamide, Manganese Gluconate, Maltodextrin, Copper Gluconate, D-Calcium Pantothenate, Pyridoxine Hydrochloride, Riboflavin, Thiamin Hydrochloride, Retinyl Acetate, Folic Acid (Pteroylmonoglutamic Acid), Potassium Iodide, Sodium Molybdate, Chromium Chloride, Sodium Selenite, Vitamin K (Phylloquinone), D-Biotin, Vitamin D3 (Cholecalciferol), Vitamin B12 (Cyanocobalamin)..

The process as defined herein is intended to cover a specific heat treatment process for the composition as claimed.

In this regard, the inventors have found that the processing of high protein, high fat compositions, especially wherein they contain added calcium and micronutrients can present difficulties during heat treatment using the methods as described in the prior art.

Products containing high levels of protein, fat, carbohydrate and micronutrients can often present processing difficulty in retort and pasteurization and/or sterilization due to:
1) High viscosity during mixing phases: poor homogeneity, difficult to pump, homogenize and heat process particularly in steam injection plate heat exchangers.
2) Aeration during mixing due to high protein level: difficult to pump from mixing tank, homogenise and heat process.
3) Product fouling in the heating stage. Furthermore, high protein in combination with high fat and micronutrients is prone to burning during heating.
4) Undesirable thickening of the product
5) Overcooked taste or flavour deterioration: due to high temperatures (UHT) or long heating times (retort)
6) Problems of low emulsion stability, and/or phase separation: due to high temperatures and long heating times.

This problem has been solved herein by employing low pH (<4.3, prefereably from about pH 3.5 to about pH 4.2) pasteurization process which allows a lower temperature (than UHT) and shorter time (than retort) so the process is more gentle on protein containing products, giving optimum product characteristics, taste and viscosity range from liquid to semi solid, particularly a semi-solid.

Preferably, a scrape surface heat exchanger in tubular system are also employed since this keeps the product moving and therefore reduces risk of protein fouling so higher protein addition possible.

Furthermore, it has also been found that by preferably choosing a protein such as an acidified whey protein as a protein source, employing antifoaming agents such as lecithin and/or MCT oil and/or silicone antifoamers - which result in the reduction of foaming and increased processability. Furthermore, it has also been found that preferably slow mixing especially during protein addition is also beneficial for the inventive process.

In addition, preferably the inclusion of calcium lactate and/or calcium acetate and/or Magnesium lactate is also beneficial since these improve the protein stability of the product.

The inventors have also found that for the process of the present invention, the package or sealable package as used herein is a bottle, pot, pouch, tear top tube and the like. A Guala pack and the like is particularly preferable since this is complementary to the viscosity of the inventive compositions. In this regard, if any other type of pack is used the product may thicken too much to be consumed directly from the pack. More specifically, since thickening of the inventive composition occurs inside said packaging this allows for higher protein addition and hence if desired a more viscous, semi solid/semi liquid inventive composition, particularly a liquid can be produced.

According to a further embodiment, uses of the inventive compositions as described herein, either as described initially or as obtained or obtainable according to the inventive process, are contemplated. The inventive composition is particularly suitable for the use in the dietary management of diseases or disorders as defined herein, such as malnutrition, malabsorption states and other conditions requiring fortification with energy, protein and micronutrients.

The term "fortification" as used herein means to supplement or add nutrients to the inventive composition, preferably during the inventive process as described herein that may be lacking in the overall diet. Such nutrients include, but are not limited to folate, vitamins A and D, and calcium.

The composition is also advantageously suitable for the treatment of various states of oncology, cystic fibrosis, elderly (dementia), neuro-disabilities, improvement of brain function or of cognitive skills, neurological diseases, neurodegenerative diseases, dysphasia, pre- and post operative surgery, ADHD (Attention Deficit Hyperactivity Disorder), GLUT-1 deficiency, Pyruvate Dehydrogenase (PDH) deficiency, phosphofructokinase (PFK) deficiency, stroke, liver disease, Cancer (renal, head and neck) or may be suitable for use in treatment of neurological diseases, such as for treating epilepsy, preferably refractory epilepsy in children. Furthermore, the present invention may be suitable for use in the treatment of Dravet Syndrome (severe myoclonic epilepsy) and Doose Syndrome (myoclonic astatic epilepsy), epileptic syndromes and seizures, myoclonic jerk, tumor, obesity, Diabetes, gut motility disorders including constipation, Gastro Intestinal, allergy, anti-aging and psychiatric disorders.

Furthermore, the inventive composition can also be used for treating a disorder or a disease treatable with the ketogenic diet and selected from neurodegenerative diseases, and improvement of brain function or of cognitive skills, Alzheimer disease/cognitive impairment, Parkinson's disease, neurological diseases, Amyotrophic lateral sclerosis, Traumatic brain injury, Hypoxic/ischemic brain injury, Autism, ADHD (Attention Deficit Hyperactivity Disorder), Depression, Headaches, Migraine Headaches, Narcolepsy, GLUT-1 deficiency, Pyruvate Dehydrogenase (PDH) deficiency, phosphofructokinase (PFK) deficiency, Glycogenosis type V (McArdle disease), Cardiac ischemia, Rett syndrome, Tuberous Sclerosis, Diabetes and Cancer (astrocytomas, prostate, gastric, renal, head and neck).

As used herein, the term "a disorder" or " a disease" refers to any derangement or abnormality of function; a morbid physical or mental state. See Dorland's Illustrated Medical Dictionary, (W.B. Saunders Co. 27th ed. 1988).

Treatment of such diseases or disorders is preferably accomplished by administering a therapeutically effective amount of a composition according to the present invention to a subject in need thereof. Preferably, such a composition is to be administered once daily, preferably twice daily, more preferably three times daily, wherein during administration preferably at least one unit or dose for administration is provided, as defined herein. Upon administration, preferably the total amount of energy to be administered per day is as defined before. As used herein, the term "subject" refers to an animal. Preferably, the animal is a mammal. A subject also refers to for example, primates (e.g., humans), cows, sheep, goats, horses, dogs, cats, rabbits, rats, mice, fish, birds and the like. In a preferred embodiment, the subject is a human. The term "therapeutically effective amount" of a composition of the present invention refers to an amount of the compound of the present invention that will elicit the biological or medical response of a subject, or ameliorate symptoms, slow or delay disease progression, or prevent a disease, etc. Preferably, such a "therapeutically effective amount" is a packaged dose or unit as obtained as described herein.

Particularly preferably, the inventive compositions as described herein, either as described initially or as obtained or obtainable according to the inventive process, are preferably suitable for use in children from the age of 1, in other words the inventive compositions typically may be not deemed suitable for use in infants. The present invention is however also suitable for use by adults.

In addition, a further embodiment the present invention is directed towards a method of treating a disease or disorder as defined herein, e.g. epilepsy, preferably refractory epilepsy in children, comprising administering a patient in need thereof a composition as defined herein, typically comprising about 4 to about 12% by weight protein, less than 5% by weight carbohydrate, such as from 0 to about 4.5% by weight carbohydrate, and about 25 to about 38% by weight fat.

The present invention also includes a method of treatment for dietary management of malnutrition, malabsorption states and other conditions requiring fortification with energy, protein and micronutrients by administering a patient in need thereof a composition as defined herein, typically comprising about 4 to about 12% by weight protein, less than 5% by weight carbohydrate, such as from 0 to about 4.5% by weight carbohydrate,and about 25 to about 38% by weight fat. Said other conditions including areas such asvarious states of oncology, cystic fibrosis, elderly (dementia), neuro-disabilities, improvement of brain function or of cognitive skills, neurological diseases, neurodegenerative diseases, dysphasia, pre- and post operative surgery, ADHD (Attention Deficit Hyperactivity Disorder), GLUT-1 deficiency, Pyruvate Dehydrogenase (PDH) deficiency, phosphofructokinase (PFK) deficiency, stroke, liver disease, Cancer (renal, head and neck) or may be suitable for use in treatment of neurological diseases, such as for treating epilepsy, preferably refractory epilepsy in children.

Furthermore, the present invention also includes a method of treatment for the treatment of Dravet Syndrome (severe myoclonic epilepsy) and Doose Syndrome (myoclonic astatic epilepsy), epileptic syndromes and seizures, myoclonic jerk, tumor, obesity, Diabetes, gut motility disorders including constipation, Gastro Intestinal, allergy, anti-aging and psychiatric disorders.

The present invention also includes a method of treatment for treating disorders treatable with the ketogenic diet and selected from neurodegenerative diseases, and improvement of brain function or of cognitive skills, Alzheimer disease/cognitive impairment, Parkinson's disease, neurological diseases, Amyotrophic lateral sclerosis, Traumatic brain injury, Hypoxic/ischemic brain injury, Autism, ADHD (Attention Deficit Hyperactivity Disorder),Depression, Headaches, Migraine Headaches, Narcolepsy, GLUT-1 deficiency, Pyruvate Dehydrogenase (PDH) deficiency, phosphofructokinase (PFK) deficiency, Glycogenosis type V, (McArdle disease), Cardiac ischemia, Rett syndrome, Tuberous Sclerosis, Diabetes and Cancer (astrocytornas, prostate, gastric, renal, head and neck), typically comprising administering a patient in need thereof a composition as defined herein, typically comprising about 4 to about 12% by weight protein, less than5% by weight carbohydrate, such as from 0 to about 4.5% by weight carbohydrate, and about 25 to about 38% by weight fat.

Preferably, the method of treatment of the present invention contains or comprises about 0 to about 23% by weight medium chain triglycerides and/or about 6 to about 35 % by weight long chain triglycerides.

Most preferably, the method of treatment of the present invention furthermore comprises micronutrients selected from vitamins, minerals and trace elements as defined herein. Alternatively, for some embodiments the method of treatment of the present invention may also not contain any micronutrients.

More preferably, the inventive method of treatment contains or comprises calcium, preferably in an amount of about 100 to about 245 mg per 100 g.

In a preferred embodiment compositions utilized in the inventive method of treatment do not employ a fibre source. Alternatively compositions utilized in the inventive method of treatment may optionally contain or comprise about 0.1 to about 2.5% by weight fibre.

It is further preferred that compositions utilized in the method of treatment as described herein contains or comprises lecithin, preferably in an amount of about 0.1 to about 2%.

It is also desirable that in the method of treatment herein, that the fat in the utilized composition contains or comprises about 2 to about 30% by weight saturates, about 1 to about 30% by weight mono-unsaturates and about 0.1 to about 5% by weight polyunsaturates.

It is also desirable that in compositions utilized in the method of treatment herein, 0.4 to 2.0% by weight of the fat is eicosapentaenoic acid (EPA).

It is furthermore particularly beneficial if in compositions utilized in the inventive method of treatment the protein employed is a whey protein, most preferably acidified whey protein isolate. It is also preferable if the protein, the fat and/or the carbohydrate is/are derived from at least two different sources.

Most preferably in the compositions utilized in the inventive method of treatment of the present invention the composition is a semi liquid/semi solid, even more preferable is a liquid.

Most preferably in the compositions utilized in the inventive method of treatment of the present invention the composition has a viscosity of about 1 to about 40,000 cP as defined herein.

Even more preferable is that in said compositions utilized in the inventive method of treatment the ratio of fat: the sum of proteins and carbohydrate is about 4:1 and/or that preferably the energy content of the composition is about 300 to about 380 kcal, preferably about 300 to about 360 kcal per 100g.

It is further preferred that in the inventive method of treatment the composition is administered once daily, preferably twice daily, more preferably three times daily, wherein preferably each dose comprises a 50 g pouch, most preferably a 100 g pouch and/or that the total amount of energy administered per day is about 300 to about 635 kcal, preferably about 300 to about 600 kcal.

Said inventive method of treatment is preferably used as a supplement.

It is further preferable that said method of treatment is preferably used as a sole source of nutrition as defined herein, most preferably wherein the total amount of energy administered per day is between about 1200 to about 2500 Kcals a day.

It is also desirable that the composition utilized in the inventive method of treatment herein does not comprise soy protein and/or comprise olive oil or canula oil and/or does not contain whole milk protein in a ratio of 80:20 casein protein to whey protein.

It is also preferred that the composition utilized in the inventive method of treatment herein employs intact proteins and particularly preferred that casein is not present in said method.

It is particularly preferable in the inventive method of treatment of the present invention that the composition, which is preferably a semi-liquid/semi-solid composition, most preferably a semi-solid composition is adjusted to a pH of less than about 4.3, preferably about 3.5 to about 4.2.

It is most preferable in the inventive method of treatment of the present invention that the composition furthermore comprises an antifoaming agent, preferably selected from lecithin and/or MCT oil, and/or silicone antifoamers more preferably in an amount of about 0.5 to about 23 % by weight.

Various embodiments of the invention have been described above. The descriptions are intended to be illustrative, not limitative. Thus, it will be apparent to one skilled in the art that certain modifications maybe made to the invention as described without departing from the scope of the claims set out below.

For example, as described herein, "preferred embodiment" means "preferred embodiment of the present invention". Likewise, as described herein, "various embodiments" and "another embodiment" means "various embodiments of the present invention" and "another embodiment of the present invention", respectively.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having". When used herein "consisting of" excludes any element, step, or ingredient not specified in the claim element. When used herein, "consisting essentially of" does not exclude materials or steps that do not materially affect the basic and novel characteristics of the claim. In each instance herein any of the terms "comprising", "consisting essentially of" and "consisting of" may be replaced with either of the other two terms.

Furthermore, percentages as described in the present invention can be interchangeably either % weight-by-weight (w/w) or % weight-by-volume (w/v).

Finally, all publications and patents cited in this disclosure are incorporated by reference in their entirety. To the extent the material incorporated by reference contradicts or is inconsistent with this specification, the specification will supersede any such material.

### Figures:

The following Figures are intended to illustrate the invention further. They are not intended to limit the subject matter of the invention thereto.

**Figure 1****:** Shows a schematic representation of the metabolism of LCTs and MCTs for digestion, absorption and transport of fats, *MG* = monoglycerol, *Chyl* = Chylomicrons, gp= α-glycerophosphate, *G1 = CIRC* general circulation.

### Examples:

The following examples are intended to illustrate the invention further. They are not intended to limit the subject matter of the invention thereto.

### Example 1: Exemplary composition of the present invention

| **Nutritional component** | **per 100g** | **Units** |
|---|---|---|
| energy (kJ) | 1340 | kJ |
| energy (kcal) | 320 | kcal |
| Protein | 8 | g |
| Carbohydrate | 0 | g |
| Fat | 32.5 | g |
| Vitamin premix | 109.7 | mg |
| Minerals | 907 | mg |
| Trace element mix | 244.9 | mg |
| Medium Chain Triglycerides | 0 | g |
| Long Chain Triglycerides | 32.5 | g |
| Linoleic acid | 3347 | mg |
| α-Linoleic acid | 72 | mg |

Notes:
Vitamin premix preferably consists of Vitamin A, Vitamin D, Vitamin E, Vitamin C, Vitamin K, thiamin, riboflavin, niacin, Vitamin B₆, folic acid, Vitamin B₁₂, biotin, pantothenic acid and choline. Other vitamins may also be present as defined herein above.

Minerals preferably consist of sodium, potassium, calcium, phosphorus and magnesium. Other minerals may also be present as defined herein above.

Trace element mix preferably consists of chloride, iron, copper, zinc, manganese, iodine molybdenum, selenium and chromium. Other trace elements may also be present as defined herein above.

The ingredients which can be used to make said composition include by example water, high oleic sunflower oil, acidified whey protein isolate obtained from Volac International Ltd (Volactive Hydrapro), calcium lactate, soya lecithin E322 (Emulpur IP), magnesium lactate, tri potassium citrate, sodium chloride, choline bitartrate, vitamin premix - , trace element mix, dipotassium phosphate, tri sodium citrate, sweetener (e.g. sucralose), flavouring, taurine JP8, L-carnitine L-tartrate and citric acid anhydrous.

The exemplary composition above was prepared according to the process of Example 2 below. Viscosity of the composition was 14757 cP, measured at 25°C using a Brookfield DV-E Viscometer (LV model).

### Example 2: Exemplary way of carrying out the process as described herein.

(Step (a))
   1. Water is added to a Jex Mixing Tank at about 20°C +/- 3°C. Mixing is preferably conducted with a twin impeller agitator.
   2. Lecithin is added to the tank, mixer speed set at about 725 rpm, mixed for about 5 minutes
   3. Acidified Whey Protein is added, mixer speed is reduced to about 435 rpm, mixed for about 30 minutes.
   4. High Oleic Sunflower Oil is added then increase mixer speed to about 580 rpm, mixed for about 5 minutes.
   5. Mixer speed is then reduced to about 435 rpm, mixed for about 30 minutes.
   6. Any further ingredients such as minerals, vitamins and trace elements are added, mixer speed remains at about 580 rpm, mixed for about 10 minutes.
   7. Flavours, sweeteners,Vitamin C and Vitamin E are then added, followed by mixing at about 725rpm for about 10 minutes.
   8. pH checked to ensure is less than about 4.3, preferably about 3.5 to about 4.2 then adjust as is necessary with citric acid.
   9. Product is left to degas on mixing speed of about 435 rpm for about 12 minutes. Optionally the product is processed at Specific Gravity of about 1.01 at 20°C +/-3°C or of about 1.05 at 20°C +/- 3°C.
(Step (b))
   10. Optionally preheating the mixture, preferably at a temperature as defined generally above in the description of the invention.
(Step (c))
   11. Product goes through inline sieve.
   12. Heated to about 86 to about 93°C for about 2 minutes.
   13. Homogenised at about 30 to about 240 bar, preferably about 80 bar to about 220 bar; more preferably about 190 to about 210 bar.
(Step (d))
   14. Packed into Guala pack.
(Step (e))
   15. Pasteurised in pouch at about 76 to about 95°C for 3 minutes +/- 30 seconds
(Step (f))
   18. Cooling bath, product must be less than 30°C at the end of cooling.
   19. Packed into boxes.

## Claims

1. A composition comprising about 4 to about 12% by weight protein, less than 5% by weight carbohydrate and about 25 to about 38% by weight fat for use in treating epilepsy, preferably refractory epilepsy in children.

2. A composition comprising about 4 to about 12% by weight protein, less than 5% by weight carbohydrate and about 25 to about 38% by weight fat for use in treating disorders treatable with the ketogenic diet selected from Alzheimer disease/cognitive impairment, Parkinson's disease, neurological diseases, Amyotrophic lateral sclerosis, Traumatic brain injury, Hypoxic/ischemic brain injury, Autism, ADHD (Attention Deficit Hyperactivity Disorder), Depression, Headaches, Narcolepsy, GLUT-1 deficiency, Pyruvate Dehydrogenase (PDH) deficiency, phosphofructokinase (PFK) deficiency, Glycogenosis type V (McArdle disease), Cardiac ischemia, Rett syndrome, Tuberous Sclerosis, and Cancer (astrocytornas, prostate, gastric, renal, head and neck)).

3. The composition of claim 1 or 2 which contains 0 to about 23% by weight medium chain triglycerides and/or about 6 to about 35 % by weight long chain triglycerides.

4. The composition according to any of the preceding claims which furthermore comprises micronutrients selected from vitamins, minerals and trace elements.

5. The composition according to any of the preceding claims further comprising calcium, preferably in an amount of about 100 to about 245 mg per 100 g.

6. The composition according to any of the preceding claims further comprising lecithin preferably in an amount of about 0.1 to about 2%.

7. The composition according to any of the preceding claims wherein the fat contains about 2 to about 30% by weight saturates, about 1 to about 30% by weight mono-unsaturates and about 0.1 to about 5% by weight polyunsaturates.

8. The composition according to any of the preceding claims wherein said protein is acidified whey protein isolate.

9. The composition according to any of the preceding claims wherein the protein, the fat and/or the carbohydrate is/are derived from at least two different sources.

10. The composition according to any of the preceding claims wherein said composition is a semi solid.

11. The composition according to any of the preceding claims which has a viscosity of about 1 to about 40,000 cP.

12. The composition according to any of the preceding claims wherein the ratio of fat:protein: carbohydrate is about 4: 1: 0.01.

13. The composition according to any of the preceding claims wherein the ratio of fat to the sum of proteins and carbohydrates is about 4:1.

14. The composition according to any of the preceding claims wherein the energy content of the composition is about 300 to about 380 kcal per 100g.

15. The composition according to any of the preceding claims wherein said composition is administered once daily, preferably twice daily, more preferably three times daily.

16. The composition according to claim 15 wherein said composition is used as a supplement in amounts of 1 to 2 x 30g, 50g or 100g pouches a day.

17. The composition according to claim 15 wherein said composition is used as a sole source of nutrition in 4 to 8 x 30g, 50g or 100g pouches a day.

18. The composition according to claim 16 wherein the total amount of energy administered per day is between about 300 to about 635 Kcal a day.

19. The composition according to claim 17 wherein the total amount of energy administered per day is between about 1200 to about 2500 Kcals a day.

20. The composition according to any of the preceding claims wherein the composition is adjusted to a pH of less than about 4.3, preferably about 3.5 to about 4.2.

21. The composition according to any of the preceding claims wherein said composition furthermore comprises an antifoaming agent, preferably selected from lecithin and/or MCT oil, more preferably in an amount of about 0.5 to about 23 % by weight.

22. A process for the preparation of a composition, preferably a ketogenic composition, comprising the following steps:
(a) Mixing about 4 to about 12% by weight protein, less than 5% by weight, preferably from 0 to 4.5 % by weight, carbohydrate and about 25 to about 38% by weight fat with water;
(b) Optionally preheating the mixture;
(c) Homogenizing the mixture obtained according to step (a) or (b) at about 86 to about 92°C, preferably at a pressure of about 30 to about 220 bar.
(d) Packaging the mixture into a package, which is preferably a sealable package;
(e) Pasteurizing the packaged mixture at about 76 to about 93°C, preferably for about 2 to about 10 minutes;
(f) Preferably cooling the pasteurized packaged mixture.

23. Composition, preferably a ketogenic composition, obtained by a process according to claim 22.

24. Composition, preferably a ketogenic composition, obtained by a process according to claim 22 for use in treating disorders treatable with the ketogenic diet selected from Alzheimer disease/cognitive impairment, Parkinson's disease, neurological diseases, Amyotrophic lateral sclerosis, Traumatic brain injury, Hypoxic/ischemic brain injury, Autism, ADHD (Attention Deficit Hyperactivity Disorder), Depression, Headaches, Narcolepsy, GLUT-1 deficiency, Pyruvate Dehydrogenase (PDH) deficiency, phosphofructokinase (PFK) deficiency, Glycogenosis type V (McArdle disease), Cardiac ischemia, Rett syndrome, Tuberous Sclerosis, and Cancer (astro-cytomas, prostate, gastric, renal, head and neck).

25. Composition obtained by a process according to claim 22 for use in treating epilepsy, preferably refractory epilepsy in children.
